# EUROPEAN PATENT APPLICATION

(11) **EP 1 745 802 A1**
(43) Date of publication of application: **24.01.2007**
(21) Application number: 05076682.3
(22) Date of filing: 20.07.2005
(51) Int. Cl.: A61K 47/48

(54) **Method of conjugating therapeutic compounds to cell targeting moieties via metal complexes**

(71) Applicant: Kreatech Biotechnology B.V., 1032 LG Amsterdam (NL)
(72) Inventor: Heetebrij, Robert Jochem, 2313 SJ Leiden (NL); Kok, Robbert Jan, 9745 DR Groningen (NL); Talman, Eduard Gerhard, 1111 TL Diemen (NL); Poelstra, Klaas, 9285 TP Buitenpost (NL); Molema, Grietje, 9718 BE Groningen (NL)
(74) Representative: Winckels, Johannes Hubertus F.

(57) **Abstract**

The present invention relates to a cell-targeting complex comprising a targeting moiety and a deliverable compound, wherein said targeting moiety and said deliverable compound are joined by means of a (transition) metal ion complex having at least a first reactive moiety for forming a coordination bond with a reactive site of said targeting moiety and having at least a second reactive moiety for forming a coordination bond with a reactive site of said deliverable compound, and wherein said deliverable compound is a therapeutic compound.

## Description

### FIELD OF THE INVENTION

The present invention is in the field of drug targeting, and relates in particular to the targeting of drugs to selected cell populations and to pharmaceutical compositions comprising cell-targeting moieties.

### BACKGROUND OF THE INVENTION

Site-specific or targeted delivery of drugs is considered a valuable tool to improve the therapeutic efficacy and to reduce the toxicity of drugs. Whereas non-targeted drug compounds typically reach their intended target cells via whole-body diffusion and passive diffusion or receptor mediated uptake over the cell membrane, targeted drugs home-in and concentrate only at the targeted tissues. Consequently, targeted drugs require smaller dosages while still allowing the drug to reach therapeutically effective levels inside the target cells. Also, the preferred lipophilic character of non-targeted drugs, which facilitates their easy passage over the cell membrane and which feature is not always in agreement with other requirements of the drug, is less relevant to targeted drugs. The targeting of drugs to specific cells is therefore a conceptually attractive method to enhance specificity, to decrease systemic toxicity and to allow for the therapeutic use of compounds that are in principle less suitable or unsuitable as systemic drugs.

In general, drug delivery technologies are aimed at altering the interaction of the drug with the *in vivo* environment and achieve that objective by conjugation of the drug with other molecules, entrapment of the drug within matrices or particles or simply by co-administration with other agents. The net result is either drug targeting or enhanced drug transport across biological barriers such that its bioavailability is improved with a reduction of the incidence of clinical side effects in subjects. Drug targeting is achieved when an alteration in the drug's bio-distribution favours drug accumulation at the desired site, which site is usually remote from the administration site.

Cell-selective delivery of drugs can, in principle, be obtained by coupling drug molecules to targeting moieties (macromolecular carriers that contain a chemical moiety that is specifically recognised by target cells in the diseased tissue). However, at present such cell-specific drug targeting preparations are only scarcely available due to some major technological hurdles. Apart from the availability of suitable drugs and targeting molecules, the linkage between the therapeutic agent and the targeting device often poses significant problems. For instance, chemically reactive groups for conventional conjugation chemistry may be absent, or chemically reactive groups may be (abundantly) present, but covalent linkage may (irreversibly) inhibit the bioactivity of the coupled therapeutic agent.

### SUMMARY OF THE INVENTION

The present invention provides for an improved coupling of therapeutic compounds to targeting groups. In a first aspect, the present invention provides a cell-targeting complex comprising a targeting moiety and a deliverable compound, wherein said targeting moiety and said deliverable compound are joined by means of a (transition) metal ion complex having at least a (first) reactive moiety for forming a coordination bond with a reactive site of said targeting moiety and having at least a (second) reactive moiety for forming a coordination bond with a reactive site of said deliverable compound, and wherein said deliverable compound is a therapeutic compound. In a preferred embodiment, the metal ion complex is a platinum complex. The platinum complex may be a trans-platinum complex or it may be a *cis-*platinum complex. The cis-platinum complex furthermore preferably comprises an inert bidentate moiety as a stabilising bridge. In yet another preferred embodiment, the (platinum) metal ion complex comprises a tridentate moiety as stabilising bridge. In another embodiment of said metal ion complexes, said complex comprises at least two (transition) metal ions, whether or not the same. The reactive sites on the targeting moiety and deliverable compound are capable of forming (coordination) bonds with the (transition) metal ion complex. In preferred embodiments the targeting moiety and/or the deliverable compound therefore comprise one or more sulphur-containing reactive sites and/or one or more nitrogen-containing reactive sites.

The targeting moiety is preferably a member of a binding pair. In preferred embodiments, the targeting moiety may be an antibody or containing parts thereof (e.g. single chain, Fab-fragments), a receptor ligand or a charge-modified or terminal sugar moiety-modified macromolecular carrier. Alternatively, the carrier may be modified with peptide moieties facilitating receptor recognition. In another preferred embodiment, the targeting moiety is a low molecular weight protein such as for instance lysozyme or any other protein. In other preferred embodiments of the cell-targeting complex of the invention, the deliverable compound is a protein, more preferably a recombinant protein. In order to facilitate bonding to the metal ion complex, the (recombinant) protein preferably comprises at least one methionine or cysteine residue as a sulphur-containing reactive site or a histidine residue as a nitrogen-containing reactive site.

In a further aspect, the present invention provides a pharmaceutical composition comprising a cell-targeting complex as described herein, and a pharmaceutically acceptable formulation for the intended application.

In yet another aspect, the present invention provides a cell-targeting complex comprising a targeting moiety and a deliverable compound, wherein said targeting moiety and said deliverable compound are joined by means of a (transition) metal ion complex having at least a first reactive moiety for forming a coordination bond with a reactive site of said targeting moiety and having at least a second reactive moiety for forming a coordination bond with a reactive site of said deliverable compound, for use as a medicament.

In a further aspect, the present invention provides the use of a (transition) metal ion complex capable of forming coordination bonds, for linking a targeting moiety to a therapeutic compound. In preferred embodiments of this aspect, the metal ion complex is a platinum complex. Again, the platinum complex may be a trans-platinum complex or it may be a cis-platinum complex. In the case of a platinum complex, comprising a stabilising bridge, these platinum complexes preferably comprise an inert bidentate or tridentate moiety as a stabilising bridge. Suitably, the targeting moiety and therapeutic compound may comprise one or more sulphur-containing reactive sites and/or one or more nitrogen containing reactive-sites for participating in the coordination bond. Metal ion complexes of the present invention contain at least one (transition) metal ion. In another preferred embodiment the metal ion complex comprises two or more transition metals for binding a targeting moiety to a therapeutic compound.

In a further aspect, the present invention provides a method of coupling a targeting moiety to a therapeutic compound comprising a (transition) metal ion complex having at least a first reactive moiety for forming a coordination bond with a reactive site of said targeting moiety and having at least a second reactive moiety for forming a coordination bond with a reactive site of said therapeutic compound and allowing said (transition) metal ion complex to form coordination bonds with said targeting moiety and said therapeutic compound. Preferred embodiments of this aspect of the invention include those embodiments as described for the cell-targeting complex and the use of the invention as described above.

In a further aspect, the present invention provides a method of targeting therapeutic compounds to selected cell populations, comprising administering to a subject in need thereof a therapeutically effective amount of a pharmaceutical composition according to the present invention.

### DESCRIPTION OF THE FIGURES

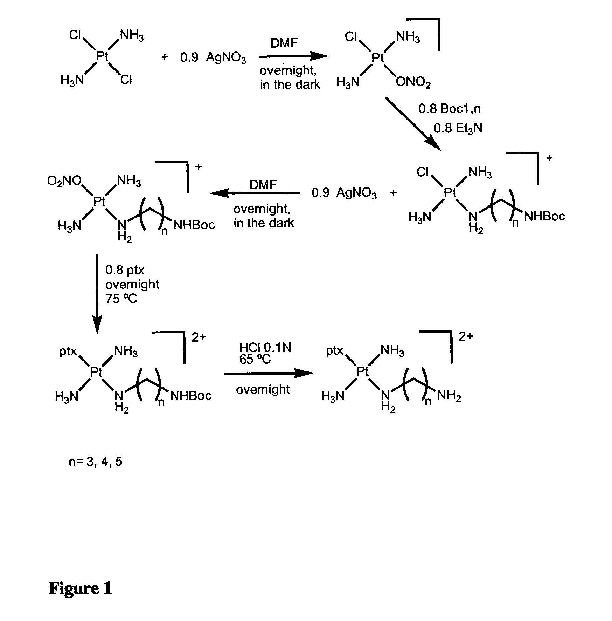

Figure 1 is a reaction scheme of the modified [Pt(ethylenediamine)dichloride] systems trans-[Pt(NH3)2(ptx)(NH2(CH)nNH2)] (n = number of C atoms in the aliphatic chain of the linkers).

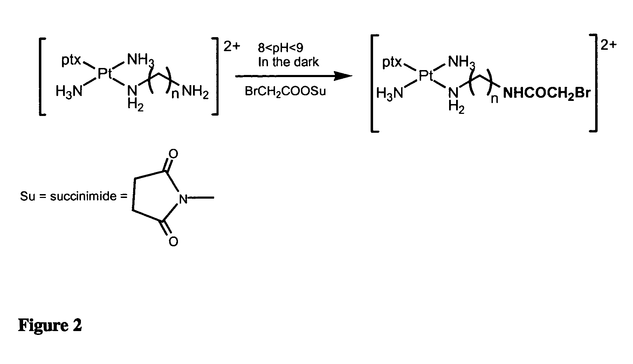

Figure 2 is a reaction scheme of the modification of the NH2 group of the linker.

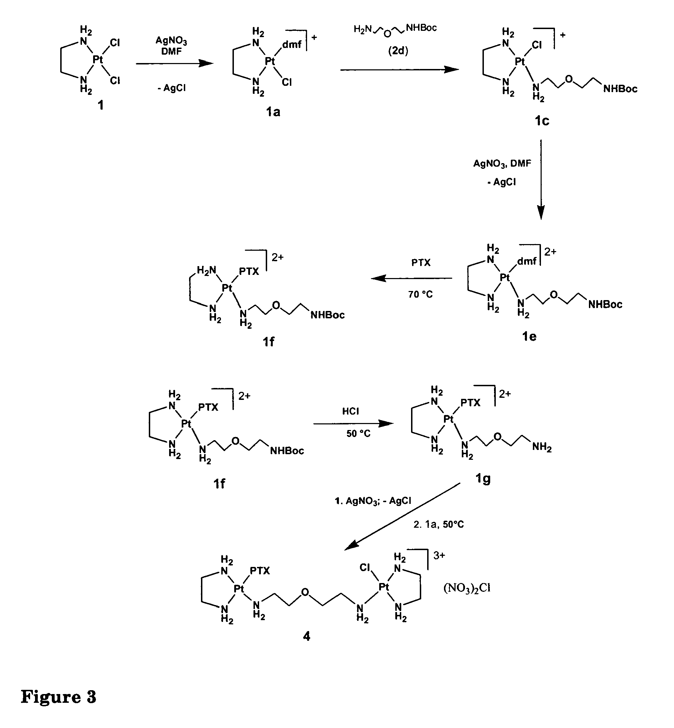

Figure 3 is a synthetic scheme for the preparation of the dinuclear conjugate 4.

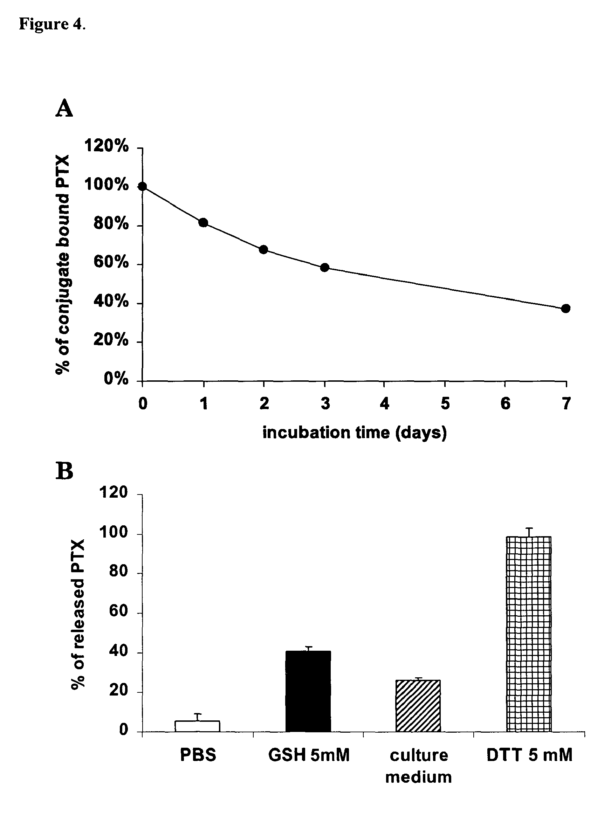

Figure 4 shows the drug release profiles of PTX-cisULS-M6PHSA conjugate. The released drug was related to the total amount of coupled PTX as determined by KSCN mediated displacement at 80°C. Panel A): Incubation of PTX-M6PHSA in serum-like conditions at 37°C for 7 days; Panel B): Release of PTX after 24h incubation with buffer, culture medium or thiol-containing competitors.

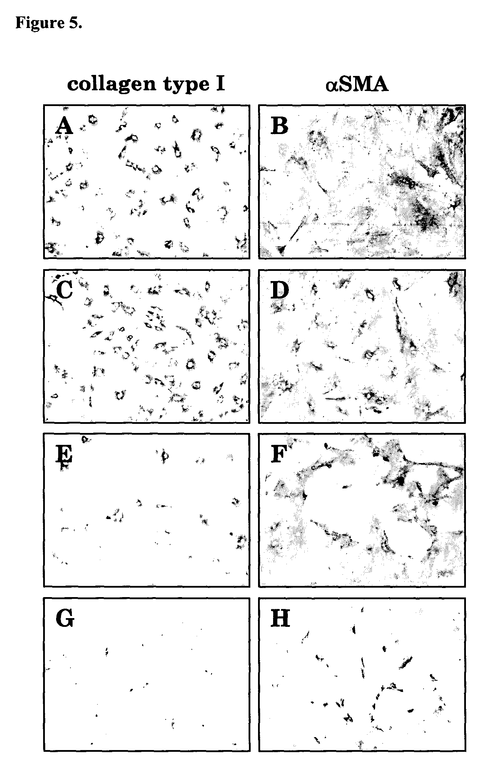

Figure 5 shows the effects of PTX-cisULS-M6PHSA on fibrosis markers collagen I and a-SMA. HSC were incubated for 24h with PTX-cisULS-HSA, PTX-cisULS-M6PHSA (both at 1 mg/ml, equivalent to 100 µM PTX) or 1 mM non-targeted PTX. Cells were stained for collagen type I or a-SMA and counterstained with hematoxillin. A,B) control cells; C,D) PTX-cisULS-HSA; E,F) PTX-cisULS-M6PHSA; G,H) non-targeted PTX. Magnification 10x.

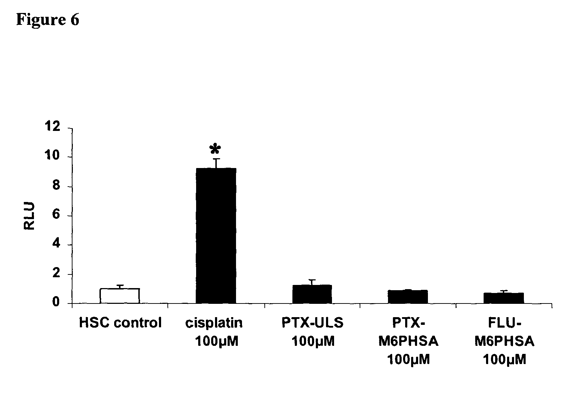

Figure 6 shows the apoptotic effects of PTX-cisULS-M6PHSA conjugates. HSC were incubated for 24h with cisplatin, PTX-ULS, PTX-cisULS-M6PHSA or fluorescein-cisULS-M6PHSA (all at a concentration equivalent to 100 µM platinum). Effect of conjugates on caspase 3/7 activity was measured using a luminescent caspase substrate and expressed relative to the signal of control HSC. RLU: relative light units, *: p<0.05 vs basal levels.

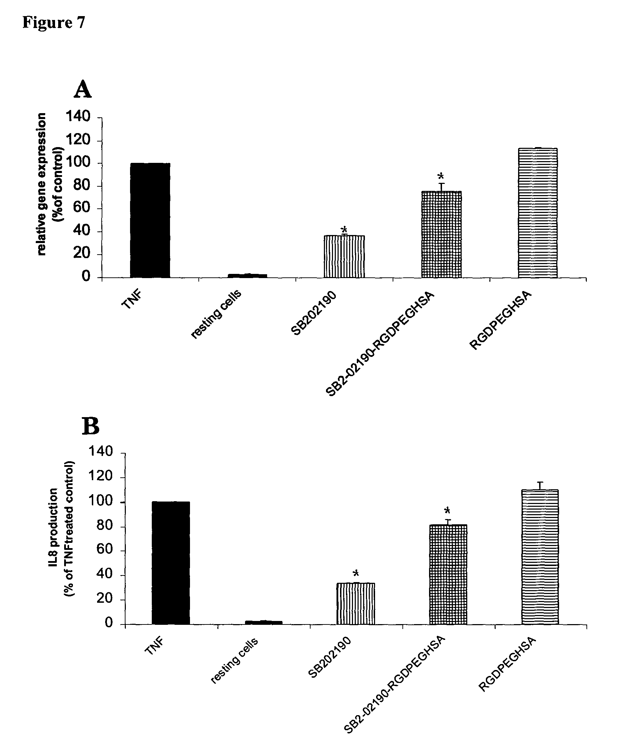

Figure 7 shows the effect of SB202190-cisULS-RGDPEG-HSA on TNFalfa-activated HUVEC. HUVEC endothelial cells were incubated for 24h with TNFalfa (10 ng/ml) to induce inflammatory events in this cell type, which was analysed at gene-expression levels of IL-8 (panel A) and IL-8 protein levels in the medium (panel B). IL-8 expression/production of TNF-treated HUVEC without inhibitor was set at 100%. The effect of free SB202190 (10 µM), the drug targeting construct SB202190-cisULS-RGDPEG-HSA (21 µg/ml of conjugate) and RGDPEG-HSA without drug (21 µg/ml) were tested by preincubating the cells for 24h, followed by addition of TNFalfa and another 24 h of incubation. *p<0.05, n=4 per treatment group.

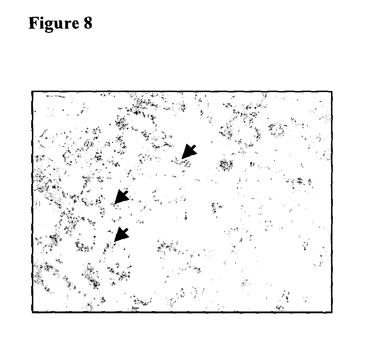

Figure 8 shows the accumulation of SB202190-cisULS-LZM in the kidney. SB202190-cisULS-LZM was administered intravenously to male Wistar rats at a dose of 16 mg/kg, after which the animal was sacrificed at 2h after administration. The presence of the conjugate within the kidney was detected by immunostaining with an anti-LZM antibody. Arrows indicate positive staining (red) in the proximal tubular cells of the kidney, which is the intended target cell of type.

### DETAILED DESCRIPTION OF THE INVENTION

### Definitions

The term "targeting moiety" as used herein refers to any molecule that can redirect, modify or improve the pharmacokinetic properties of a drug or other type of deliverable compound. In a preferred embodiment, the targeting moiety represents a compound or part of a molecule which functionally interacts with a binding site on the surface of the cell. Thus, the targeting moiety provides specificity for or binding affinity for one or more cell types. The molecule on the cell which is targeted by the targeting moiety can be any selected target, such as for instance a cell surface receptor. Targeting moieties include, but are not limited to, antibodies, antibody fragments, endogenous or non-endogenous ligands for a cell-surface receptor, antigen-binding proteins, viral surface components, proteins that bind viral surface components, growth factors, lectins, carbohydrates, fatty acids or other hydrophobic substituents, peptides and peptidomimetic molecules. Targeting moieties may in turn be complexed with macromolecules or particles to yield multivalent structures.

In another preferred embodiment, the targeting moiety does not bind to target receptors, but affects the body distribution by altering the clearance route or rate of the deliverable compound via other principles. This class of targeting moieties includes, but is not limited to, polyethylenglycol (PEG) polymers. Another non-limiting example of such a type of targeting moiety is a polymeric biomaterial which functions as depot that is releasing the coupled deliverable compound. Such a device may circulate in the blood stream or may be implanted in another part of the body, for instance subcutaneously.

The term "deliverable compound" as used herein refers to a compound which is carried or transported by the targeting moiety to the surface and/or interior of a cell. In aspects of the present invention, the deliverable compound is a therapeutic or diagnostic compound. The terms "deliverable compound and "therapeutic compound" and "drug" are used interchangeably herein and represent any agent that can be applied for therapeutic or diagnostic use. Also, the therapeutic compound may be a prodrug, which requires (bio)chemical modification to reach its active form.

In a preferred embodiment, the therapeutic compound is an organic molecule that contains an aromatic nitrogen or a sulphur atom which can form a coordination bond with the linker. The therapeutic compound may also contain a prodrug moiety that contains an aromatic nitrogen or a sulphur atom which can form a coordination bond with the linker. In another preferred embodiment, the therapeutic compound is a therapeutically active peptide or protein.

The term "reactive moiety" refers to a chemical group, a free orbital reactive site or a ligand of the metal ion complex compound that is capable of forming a bond with a reactive site of either a targeting moiety or a deliverable compound.

The term "ligands" as used herein refers to a molecule that binds to another molecule, used especially to refer to a small molecule that binds specifically to a larger molecule, e.g., an antigen binding to an antibody, or ligand binding to a receptor, or a substrate or allosteric effector binding to an enzyme. The term "ligand" is also used to indicate molecules that donate or accept a pair of electrons to form a coordinate bond with the central metal atom of a coordination complex. Thus meaning of the term ligand is context-related.

The term "(transition) metal ion complex" as used herein refers to the linker system used to couple the targeting moiety to the deliverable compound. A characteristic of such complexes is the presence therein of coordination bonds. Metal ions suitable for use in a (transition) metal ion complex used in the present invention are metal ions capable of forming coordination bonds with ligands. Thus, transition metals such as Pt, Ru, Cu, Zn, Ni, Pd, Os and Cd are amenable for use in the present invention. The (transition) metal ion complex of the present invention consists of a central metal ion bound to a number of other molecules, termed ligands. The nature of the chemical bond formed between ligand and metal can be thought of as involving the donation of a pair of electrons present on the ligand molecule or, in molecular orbital terms, as a molecular orbital formed by combining a filled orbital of the ligand with a vacant orbital of the metal. Those atoms in the ligand molecule that are directly involved in forming a chemical bond to the metal ion are therefore termed the donor atoms, generally comprising elements of Groups V and VI of the periodic table, with nitrogen, oxygen, sulfur, phosphorus and arsenic being those most commonly employed.

Molecules that contain two or more donor atoms capable of binding to a single metal ion are termed chelating agents, or chelators, and the corresponding metal complexes are called chelates. The number of donor atoms present in a given chelator is termed the denticity of that chelator, ligands possessing two donor sites being called bidentate, those with three donor sites, tridentate, and so forth. In general, the higher the denticity of a chelator the more stable are the chelates formed, up to the point at which the denticity of the chelator matches the maximum coordination number attainable by the particular metal ion of interest. The maximum coordination number of a given metal ion in a given oxidation state is an intrinsic property of that metal, reflecting the number of vacant orbitals and, hence, the number of chemical bonds it is able to form with ligand donor atoms. When all of the available vacant orbitals have been used to form bonds to donor atoms in the ligand or ligands, the metal is said to be coordinatively saturated.

For any given metal ion, useful ligands according to the present invention may be selected by one skilled in the art, employing the following criteria. Ligands must possess donor atoms (or sets of donor atoms in the case of chelating ligands) that favor binding to the target metal ion. The general preference of any given metal ion for particular donor atoms (generally selected from the group consisting of carboxylic, phenolic, or ether oxygen atoms, amine, imine, or aromatic nitrogen atoms and charged or neutral sulfur atoms) is well known in the art.

The ligands must also offer the prospect of forming ligand-metal linkages that are likely to display appropriate stability in vivo. That is, the ligand-metal linkage does not dissociate during the time required to transport the therapeutic compound to the target cell. In addition, but depending on the intended application for any given deliverable compound, the ligand-metal linkage must offer the prospect of drug-release inside the target cell or at the target site. For many metals of interest there exists considerable art relating to the in vivo stability of particular ligand-metal linkages, which may be used to guide ligand-metal selection.

Within the scope of the present invention, there are many useful ligands that have been shown to coordinately bond to metal ions; they may either be mono, bi, or tridentate, depending on the number of sites that the ligand binds to the metal ion.

The above described ligands may be bifunctional. A bifunctional ligand is a molecule that contains, in addition to at least one metal binding site (donor atom), a second reactive moiety through which the ligand may be linked to, for example, a protein, without significantly affecting the metal-binding properties of the ligand.

The present invention provides for a novel type of linker system or linker in which the deliverable compound is joined by means of one or more coordination bonds with the targeting moiety. Several important problems in the field of designing targeted drugs have motivated us to create such a novel transition metal based linker technology and the "cell targeting complexes" that result from combining such linkers with deliverable compounds and targeting moieties.

In this respect, many small organic drugs do not have functional groups that can be used for the chemical linkage technologies (e.g. ester linkage) that are commonly applied to couple drugs to a targeting moiety. Thus, the existing (*covalent*) linkage methods are insufficient to prepare a cell targeting complex with appropriate stability of the drug-carrier linkage in vivo as described above. In this respect, the linkage system is very important and should provide for bonding that prevents premature release of the drug from the carrier in the circulation, while not being too stable, so that the drug remains inactive even when accumulated at/or in the target site. The application of a novel linker technology as described in the present invention creates the opportunity to couple drug molecules via one or more coordination bonds with the (transition) metal complex. One major advantage of the described linker system is that it allows for the development of cell targeting complexes with drugs that could not be produced previously due to the lack of coupling technology.

Also, in the event of a coordinative bond between the linker and the deliverable compound, e.g. a drug, the bond may be reversed, especially when small electron rich materials are present, e.g. in vivo gluthathione, in vitro potassium thiocyanate. The structure of the deliverable compound is not affected or changed upon binding and release.

Also, natural occurring linker binding sites, e.g. nitrogen and sulphur, in the deliverable compound eliminates the need to modify chemically the deliverable compound.

Furthermore, the coupling of therapeutic proteins (such as anti-inflammatory cytokines) to targeting moieties often causes the formation of undefined complexes of variable size and composition. Partly, this problem originates from the chemistry applied in the conjugation of the targeting moiety to the therapeutic protein. Many covalent linkers are directed to reactive sites like terminal amino groups of lysyl residues that are abundantly present within the protein. A linker system that reacts at more specific locations in the protein, as defined by the distribution of cysteine, methione and/or histidine residues within the protein, will yield better defined cell targeting complexes. In a preferred embodiment, the cell targeting complex has been prepared by reacting a recombinant protein displaying a "methionine start codon" or a "histidine tag" sequence, or a combination of these sequences, that both can react with the transition metal linker.

A cell-targeting complex of the present invention may have various forms or constitutions wherein the targeting moiety and a therapeutic compound are joined by means of a (transition) metal ion containing linker system. In (transition) metal ion complexes, platinum is the preferred metal ion.

A cell-targeting complex of the invention may be represented by the formula:

DC-M-TM

wherein
- DC is the deliverable compound,
- TM is the targeting moiety, and
- M is a (transition) metal ion linker system.

The deliverable compound and the targeting moiety may be coupled directly to the (transition) metal ion linker system by means of a coordination bond. Also, the deliverable compound or the targeting moiety may be coupled to an inert multiple dentate moiety or stabilising bridge in a metal ion complex. For instance, the deliverable compound may be coupled to the ethylenediamine stabilising bridge of a cis-platinum complex. The (transition) metal atom then functions as a reactive moiety for coupling to the targeting moiety only, and not (directly) to the deliverable compound.

A cell-targeting complex of the present invention may comprise a spacer between the linker and the deliverable compound and/or targeting moiety. Such a cell-targeting complex may be represented by the formula:

DC-X₁-M-X₂-TM

wherein
- DC, TM and M are as defined above, and
- X₁ is a spacer group or prodrug coupled to the (transition) metal atom by means of a coordination bond, and wherein X₁ is coupled by other chemical bonding (e.g. by covalent bond) to the deliverable compound. X₁ may very suitably be a spacer comprising a (transition) metal or a (enzyme responsive) cleavage site to allow for release of the therapeutic compound from the metal ion complex, and
- X₂ is a spacer group between the metal atom and the targeting moiety. X₂ may very suitably be a spacer comprising a (transition) metal or a (enzyme responsive) cleavage site to allow for release of the deliverable compound and metal ion complex from the targeting moiety.

Suitable types of spacer groups are molecules that allow for an appropriate distance between the metal ion complex and another functional group and also allow further modification of the linker complex. Preferred linkers are [NH₂-(CH₂)ₙ-NH₂] wherein n varies between 1 and 10, preferably between 1 and 6. For instance, the spacer diaminohexane [NH₂-(CH₂)₆-NH₂] has been proven very useful in preferred embodiments of this invention. Other preferred linkers are [NH₂-(CH₂)ₙ-A-(CH2)ₙ-NH₂] wherein A is a heteroatom, preferable N or O, and n varying between 1 and 5. Yet, another preferred type of spacer is NH₂-(CH₂)ₙ-NHR wherein n can be any number between 1 and 6, and R may be independently chosen to be C(O)CH₂Br, CO-(CH₂)ₘ-(C₄H₂NO₂) wherein m is 1 - 10 preferentially 5, CO-(C₆H₁₀)-CH₂-(C₄H₂NO₂), or CO-(CH₂)₂-NH₂-CO-CH₂-Br. Another preferred type of spacer is NH₂-(CH₂)ₙ-NH-CO-(CH₂)ₙ-mPEG wherein n varies between 1 and 6 and mPEG represents polyethyleneglycol whether or not having -(C₄H₂NO₂) bond thereto.

Also, the linker may be attached to - or being part of - the stabilizing bridge of the (transition) metal ion complex. Preferred structures are -(CH₂)ₙ-NHR wherein n can be any number varying from 1 to 10, preferentially 6, and R may be independently chosen to be C(O)CH₂Br, CO-(CH₂)ₘ-(C₄H₂NO₂) wherein m is 1 - 10 preferentially 5, CO-(C₆H₁₀)-CH₂-(C₄H₂NO₂), CO-(CH₂)₂-NH₂-CO-CH₂-Br, or -CO-(CH₂)ₙ-mPEG wherein n varies between 1 and 6 and mPEG represents polyethyleneglycol whether or not having -(C₄H₂NO₂) bond thereto.

A cell-targeting complex of the present invention may comprise more then one (transition) metal ion linker system. A (transition) metal ion complex that contains two (transition) metal ions is referred to as a dinuclear species. A dinuclear species may be represented by the formula:

M₁-A-M₂

Wherein:
- M₁ and M₂ are (transition) metal ion linkers as defined above;
- A is an aliphatic chain whether or not containing a heteroatom.

A therapeutic compound or targeting moiety linked to a platinum complex may be referred to as a Pt-S adduct (when attached to a sulphur containing reactive site), to a Pt-N adduct (when attached to a nitrogen containing reactive site), or in general to a Pt-adduct.

A sulphur containing reactive site may hereafter be referred to as a S-reactive site, and a nitrogen containing reactive site may hereafter be referred to as N-reactive site.

Methods using platinum complexes to label bio-organic molecules have been contemplated for a very long time. Platinum complexes may react with a variety of reactive moieties on biomolecules and various types of detectable marker moieties are known to have been adhered to ionic platinum.

The use of a cis-platinum complex for this purpose has for instance been described in WO96/35696, which discloses a method for linking bio-organic molecules and markers through cis-platinum complexes. In these complexes, two co-ordination sites are occupied by either ends of a stabilising bridge, such as an ethylene diamine group. Cis-platinum complexes are suitable for linking labels to several kinds of bio-organic molecules, such as peptides, polypeptides, proteins, and nucleic acids. Methods using *trans-*platinum complexes have also been reported (EP 0 870 770) as suitable for labeling a variety of bio-organic molecules and linking of labels thereto.

The present inventors have found that a particular advantage of the use of a (transition) metal ion complex as a linker between a deliverable compound and a targeting moiety in targeted drugs is that (transition) metal ion complexes provide for bonds that are strong enough to allow the deliverable compound to be transported to various tissues in vivo while remaining coupled to a targeting moiety. The reactivity of platinum complexes towards a variety of reactive sites in drug molecules is a benefit in the application as a drug linking system, since it allows straightforward conjugation reactions that result in quantitative yields of the desired products.

A further advantage of the use of (transition) metal ion complexes is that, depending on the reactive moieties used, such complexes may support the coupling of a wide variety of biologically active compounds to targeting molecules, whereby the chemistry of the targeting moieties as well as of the therapeutic compounds can vary greatly.

Another advantage is that targeting moieties or deliverable compounds may be linked via a suitable (transition) metal ion complex at histidine (His), methionine (Met) and/or cysteine (Cys) residues within protein or peptide chains. This provides for the possibility to link to different and multiple side groups in a protein or peptide chain, which may for instance allow for the linking of several deliverable compounds to one targeting moiety or vice versa. Furthermore, the linkage of drugs or targeting moieties groups via the metal ion complex to these amino acid residues will allow concomitant derivatization of lysine side chains by a conventional derivatization strategy, without interference of the two reactions. Thus, the coupling of either substituent to the core protein or peptide will not lower the reaction efficiency, as is observed in conventional strategies that aim at lysine residues with both types of reactions.

The specificity of the coupling reaction with (transition) metal ion complexes may be controlled such as to discriminate between coupling to sulphur containing reactive sites and nitrogen containing reactive sites in a targeting moiety or deliverable compound. Therefore, by using the (transition) metal ion linker according to the present invention one can direct the coupling of a targeting moiety or deliverable compound towards a specified reactive site within a targeting moiety or deliverable compound.

Platinum is a preferred metal ion in (transition) metal ion complexes.

Examples of preferred platinum complexes suitable for use in a method of the present invention are cis- or trans-platinum complexes of the formula [Pt(II)(X₃)(X₄)(T)(D)] or a cis-platinum complex of the formula [Pt(II)(X₅)(T)(D)].

Herein, Pt represents platinum (Pt), T and D represent the same or different reactive moieties, that participate in the reaction with a targeting moiety (T) or a deliverable compound (D), respectively. Another possibility is that the substituents at position T and D will be replaced by another ligand to form the complex with targeting moiety or a deliverable compound. The entities, X₃ and X₄ represent the same or different inert moieties, and X₅ represents an inert moiety that may act as a stabilising bridge such as a multiple dentate ligand, e.g. a bidentate ligand. A preferred group of bidentate ligands are aliphatic diamine bridges and diamine cyclo bridges, all sufficiently known by persons skilled in the art. An overview of preferred tridentates is given in patent application EP 04078328.4 which are incorporated herein by reference.

A structural representation of some examples of such platinum complexes is shown below:

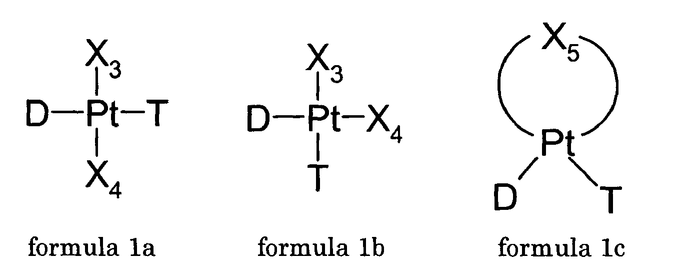

A platinum(II) complex, for use in a method of the invention can be prepared via any method known in the art. References can for example be found in Reedijk et al. (Structure and Bonding, 67, pp. 53-89, 1987). The preparation of some *trans-* platinum complexes is disclosed in EP 0 870 770. Further preparation methods can be found in WO 96/35696 and WO 98/15564. Methods described in any of these publications are incorporated herein by reference. In a preferred embodiment of the invention platinum complexes are prepared according to the spacer - *tert* butoxycarbonyl / NHS - pathway. In this approach, one of the ligands at the metal ion consists of a spacer molecule that contains an amino group distant from the metal ion that can be further derivatized for the purpose of conjugating either deliverable compound or targeting moiety.

Reactive moieties (T D) of a platinum complex that will be replaced by targeting moiety and deliverable compound are preferably good leaving ligands. A platinum complex, wherein T and/or D are independently chosen from the group of Cl⁻, NO₃⁻, HCO₃⁻, CO₃⁻, SO₃²⁻, ZSO₃⁻, I⁻, Br⁻, F⁻, acetate, carboxylate, phosphate, ethylnitrate, oxalate, citrate, a phosphonate, ZO-, and water has been found to be particularly suitable for use in a method according to the invention. Z is defined herein as a hydrogen moiety or an alkyl or aryl group having from 1 to 10 carbon atoms. Of these ligands, Cl⁻ and NO₃⁻ are most preferred.

Any type of inert moiety X₅ may be chosen. Inert as used herein indicates that the moiety remains attached to the platinum complex during the linking process. A platinum complex comprising one or two inert moieties chosen from the group of NH₃, NH₂R, NHRR', NRR'R" groups, wherein R, R' and R" preferably represent an alkyl group having from 1 to 6 carbon atoms has been found to be particularly suitable for use in a method of the present invention. H₂NCH₃ is a particularly preferred inert moiety. An alkyl diamine, wherein the alkylgroup has 2 to 6 carbon atoms is a preferred bidentate inert moiety in a cis-platinum complex (*e.g.* X5 in formula 1c). In a particularly preferred embodiment X5 represents ethylene diamine.

Particularly preferred platinum complexes for use in a method of the present invention include cis[Pt(en)Cl₂], cis[Pt(en)Cl(NO₃)], cis[Pt(en)(NO₃)₂], trans[Pt(NH₃)₂Cl₂], trans[Pt(NH₃)₂Cl(NO₃)], and trans[Pt(NH₃)₂(NO₃)₂]; in these formulas, en indicates an ethylenediamine moiety.

In principle, any type of reactive site of a targeting moiety or deliverable compound may be used as coupling site in a method of the invention. Preferred nitrogen containing reactive sites include reactive sites comprising a primary amine, a secondary amine, a tertiary amine, an aromatic amine, an amide, an imide, an imine, an iminoether, or an azide. Preferred sulphur containing reactive sites include reactive sites comprising a thiol, a thioether, a sulfide, a disulfide, a thioamide, a thion, a dithiocarbamate. Examples of targeting moieties or deliverable compounds that can be coupled to the platinum-complex linker include organic drug molecules, proteins, amino acids peptides, oligopeptides, polypeptides, immunoglobulins, enzymes, synzymes, phospholipids, glycoproteins, nucleic acids, nucleosides, nucleotides, oligonucleotides, polynucleotides, peptide nucleic acids, peptide nucleic acid oligomers, peptide nucleic acid polymers, amines, aminoglycosides, nucleopeptides, glycopeptides and sugars, as well as combinations of the molecules listed above and any organic, inorganic or biological molecule that can be used to mimic these structures.

Proteinaceous deliverable compounds and targeting moieties may be facilitated with (enzyme responsive) cleavage sites by using recombinant protein technology. However, in a preferred embodiment the drug release facility is included in the linkage system. In another preferred embodiment the cleavage site is degradable by enzymes that are specifically present within the target area (metalloproteinases, plasmin or lysosomal proteases). In this way, additional site-specificity is created to cause release of pharmacologically active compounds only in or at the target site.

The deliverable compound may be any compound exerting therapeutic effect or having a diagnostic purpose. Non-limiting examples of deliverable compounds amenable for use in the present invention may be either proteinaceous macromolecules, or smaller chemical or nature-derived drug molecules. The therapeutic compound may for instance be a bioactive protein. Molecular biology has realised the possibility to design and produce recombinant proteins that can be used as a drug. Such proteins, like for instance cytokines and growth factors, are mediators with potent biological activities. However, therapeutic applications of cytokines are often limited because of the following considerations. Firstly, these potent mediators generally display multiple biological activities, that can also be harmful to the subject. The equipment with a targeting moiety may improve the organ and cellular selectivity of the compound, and thereby greatly improve its therapeutic profile. Secondly, the plasma half-life of cytokines and many other recombinant proteins is generally short because of rapid elimination. Again, the coupling to a targeting moiety to the protein in order to affect its pharmacokinetics and/or pursue cell-specific delivery may provide a solution for this problem. In addition, targeted delivery of recombinant proteins may allow the usage of lower doses, which can facilitate the successful application of therapeutic proteins that are produced in low yield. Successful drug targeting strategies may therefore open the application of a spectrum of therapeutic proteins.

Bio-active proteins and small drugs may in principle be coupled to a targeting moiety via a stable bond that is resistant to degradation, or by a bond that is cleaved within or outside the target cell resulting in the release of the pharmacological active compound from the carrier. The present invention allows for both types of bonds, for instance through modification of the coordination (platinum) compounds.

Furthermore, a stable linkage between a protein and a targeting moiety may be realized by using a recombinant form of the protein that has been provided with a HIS-tag, i.e. a stretch of amino acid (histidine) residues attached to the protein molecule that is commonly used as an aid in its purification. The presence of both methionine residues and histidine residues in the HIS-tag make it amenable to react the metal ion linker to the HIS-tag, which may prove beneficial with respect to the therapeutic activity of the resulting complex. Furthermore, the metal ion linker may also be reacted with preference to methionine start codons of recombinant proteins that do not have a HIS-tag. This approach has wide commercial applicability because with this universal technology all recombinant protein drugs can be efficiently linked to targeting moieties by a method of the present invention.

Therapeutic compounds in the form of recombinant proteins may be produced by standard recombinant techniques. For instance, recombinant vectors encoding for the desired protein with a HIS-tag and optionally a cleavage site may be constructed by known methods.

A coupling method of the present invention comprises the step of allowing the (transition) metal ion complex to form coordination bonds with the targeting moiety and the deliverable compound.

Any order in which the bonds are formed is suitable. For instance, the (transition) metal ion complex may first form coordination bonds with the targeting moiety and, the resulting conjugate may subsequently be brought into contact with the deliverable compound so as to allow the formation of coordination bonds between the metal ion of the targeting moiety―metal conjugate and the deliverable compound. Also, all compounds may be brought together and the formation of the various bonds may commence simultaneously.

The bonds between the metal and deliverable compound and between the metal and the targeting moiety may be the same or different. For instance, the deliverable compound-metal bond may comprise a Pt-S adduct, while the targeting moiety-metal bond may comprise of a Pt-N adduct, or vice versa. Other types of bonds which will form coordination bonds with the metal ion may be selected by one skilled in the art.

The reaction parameters for the bonding reaction(s) may include the choice for a specific pH value. The pH as used herein should be interpreted as the pH value in water at 20 °C. In general, the formation of Pt-S adducts is pH independent whereas formation of Pt-N adducts is pH dependent. In a preferred embodiment one or more S-reactive sites are selectively labelled over one or more nitrogen containing sites by making use of the pH.

As a guideline, one may choose the pH of the bonding reaction at a pH below the lowest pKa of any of a deliverable compound or targeting moiety N-reactive sites that should not be bonded, allowing preferential bonding to S-reactive sites. As the skilled professional will understand other factors, besides pKa, may play a role in the bonding reaction. In general, S-reactive sites are preferentially labelled over N-reactive sites at acidic pH. Therefore, the pH may be used for preferential binding of S based ligands over N based ligands.

In theory, the formation of a Pt-S adducts is a one step process. A reactive group leaves the metal ion complex upon S donating an electron pair to metal ion. This process, the direct conversion metal ion-X into metal ion-S, is believed to be pH independent. On the other hand, N donors require replacement of a reactive group of the metal ion complex by oxygen prior to N substitution. First, metal ion-X becomes metal ion-O and eventual metal ion-N. This is a two step scheme in which the first step can be controlled by changing pH. Factors influencing pH of a solution may therefore interfere with Pt-N adduct formation. The above described metal ion bonding relates in particular to Pt-ion coordination bonding.

The presence of ions may also be used to control the selectivity of the metal ion (preferably platinum) complex for forming coordination bonds with N-reactive sites. In an embodiment one or more leaving ligands, preferably anionic moieties, are used in the inhibition of the coupling of a metal ion complex to a N-reactive site, in order to enhance preferential coupling to an S-reactive site. Examples of such leaving ligands include Cl-, NO₃-, HCO₃-, CO₃²-, ZSO₃-, SO₃-, I-, Br-, F-, acetate, carboxylate, phosphate, ethylnitrate, oxalate, citrate, a phosphonate, ZO-, and water. Z is defined herein as a hydrogen moiety or an alkyl or aryl group having from 1 to 10 carbon atoms. Particularly good results have been achieved by using salts comprising an anionic moiety, of which chloride and nitrate is particularly preferred. The counter ions are preferably alkali cations, alkali earth cations or cations also used to direct the bond formation. In a preferred embodiment the total ionic strength of said anionic moieties used in the inhibition of bonding to a N-reactive site is at least 0.1 mol/l. More preferably the total ionic strength is in the range of 0.1 to 0.5 mol/l.

The presence of other (transition) metal ions in the reaction mixture, may also be used for selection of the reactive site to be labelled. In particular several (transition) metal ions have been found suitable to prevent or slow down bonding to an S-reactive site or to make a bonded Pt-S adduct labile, so that effectively N-reactive sites are preferentially bonded over S-reactive sites. Within a method according to the invention it is also possible to direct the bonding reaction by making use of geometrical isomers of a metal ion complex - *e.g.* a *cis*-platinum complex and a *trans*-platinum complex, - such that the metal ion complex is specifically bonded to either a sulphur containing reactive site or to a nitrogen containing reactive site.

This form of preferential bonding of targeting moieties or deliverable compounds may hold specific advantages when specific bonds between the various components of the complex of the invention are required and may thus be used to control the bonding of the ion metal complex to either the targeting moiety or the deliverable compound when the coupling reaction is performed in a reaction mixture comprising both the targeting moieties and deliverable compounds.

In addition to the parameters as mentioned above a method according to the invention may further be fine tuned by parameters such as temperature, preferably varied in the range between 0 °C and 120 °C, more preferably in the range between 20 °C and 70 °C; reaction time, commonly in the range between 1 min and 48 hours, preferably in the range between 10 min and 24 hours, more preferably in the range between 25 min and 15 hours; concentration of the reagents, molar ratio of the reagents, overall net charge of the metal ion complex, and the like. These parameters may be adjusted depending upon the particular application in any way known in the art. The overall net charge of the metal ion complex, affects the specificity of metal ion-N adduct formation, for example, the specificity of Pt-N adduct formation in histidine at neutral pH. Neutral Pt-complexes form Pt-N adducts whereas positively charged platinum complexes do not. Positively charged Pt complexes display differential bonding towards N adducts above the isoelectric point of the peptide, protein, and the like. Apart from allowing the selective bonding to N-reactive sites over S-reactive sites or vice versa, a method according to the present invention also makes it possible to differentiate between distinct N-reactive sites or distinct S-reactive sites, by choosing the correct conditions, such as described in European patent application EP 1 262 778.

Purification, quality and stability of the conjugates may be performed and assessed using standard techniques. For instance, binding to and uptake of conjugates in target cells may be studied *in-vitro* using radiolabelled products or with immunohistochemical techniques. Suitable *in-vitro* test systems may for instance include cells and organ slices. Organ and cellular distribution of the developed complexes may for instance be studied in healthy animals and in experimental disease models (i.e. a rat or mouse models).

Once the cell-targeting complexes have been prepared, they may be combined with pharmaceutically acceptable carriers to form a pharmaceutical composition. As would be appreciated by one of skill in this art, the carriers may be chosen based on the route of administration as described below, the location of the target tissue, the drug being delivered, the time course of delivery of the drug, etc.

It is also an object of the invention to provide a method of targeting deliverable compounds to selected cell populations within the body. These and other objects can be addressed by providing a pharmaceutical composition comprising a cell-targeting complex according to the present invention. In a preferred embodiment the composition comprises a pharmaceutically acceptable carrier admixed with the cell-targeting complex of the invention. As used herein, the term "pharmaceutically acceptable carrier" means an inert solid, semi-solid or liquid filler, diluent, encapsulating material or formulation auxiliary of any type. A "pharmaceutically acceptable" carrier is one that is suitable for use with humans and/or animals without undue adverse side effects (such as toxicity, irritation, and allergic response) commensurate with a reasonable benefit/risk ratio. Such materials are pharmaceutically acceptable in that they are nontoxic, do not interfere with drug delivery, and are not for any other reasons biologically or otherwise undesirable. Remington's Pharmaceutical Sciences, 20th edition, Gennaro AR (ed), Mack Publishing Company, Easton, PA, 2000 discloses various carriers used in formulating pharmaceutical compositions and known techniques for the preparation thereof. Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols such as propylene glycol; esters such as ethyl oleate and ethyl laurate; agar; detergents such as TWEEN 80 ; buffering agents such as magnesium hydroxide and aluminum hydroxide; alginic acid; pyrogen-free water; isotonic saline; Ringer's solution; ethyl alcohol; and phosphate buffer solutions, as well as other nontoxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

A method of targeting deliverable compounds to selected cell populations comprises administering to a subject in need thereof a therapeutically effective amount of a pharmaceutical composition according to the invention. As used herein, "effective amount" means an amount of a drug or pharmacologically active compound that is nontoxic but sufficient to provide the desired local or systemic effect and performance at a reasonable benefit/risk ratio attending any medical treatment. As used herein, "administering" and similar terms mean delivering the composition to the individual being treated such that the composition is capable of reaching the intended target site or the systemic circulation. The composition is preferably administered to the individual by systemic administration, typically by intravenous, subcutaneous, intraperitoneal or intramuscular administration. Injectables for such use can be prepared in conventional forms, either as a liquid solution or suspension or in a solid form suitable for preparation as a solution or suspension in a liquid prior to injection, or as an emulsion. Suitable excipients or carriers include, for example, water, saline, dextrose, glycerol, ethanol, and the like; and if desired, minor amounts of auxiliary substances such as wetting or emulsifying agents, buffers, and the like can be added. Other carriers can be used and are well known in the art.

The pharmaceutical compositions of this invention can be administered to a subject by any means known in the art including oral and parenteral routes. The term "subject", as used herein, refers to humans as well as non-humans, including, for example, mammals, birds, reptiles, amphibians and fish. Preferably, the non-humans are mammals (e. g. , a rodent, a mouse, a rat, a rabbit, a monkey, a dog, a cat, a primate, or a pig). In certain embodiments parenteral routes are preferred since they avoid contact with the digestive enzymes that are found in the gastrointestinal tract.

The pharmaceutical compositions may be administered by injection (e.g. , intravenous, subcutaneous or intramuscular, intraperitoneal injection), rectally, vaginally, topically (as by powders, creams, ointments, or drops), or by inhalation (as by sprays).

Injectable preparations, for example, sterile injectable aqueous or oleaginous suspensions may be formulated according to the known art using suitable dispersing or wetting agents and suspending agents. The sterile injectable preparation may also be a sterile injectable solution, suspension, or emulsion in a nontoxic parenterally acceptable diluent or solvent, for example, as a solution in 1,3-butanediol. Among the acceptable vehicles and solvents that may be employed are water, Ringer's solution, U. S. P. and isotonic sodium chloride solution. In addition, sterile, fixed oils are conventionally employed as a solvent or suspending medium. For this purpose any bland fixed oil can be employed including synthetic mono-or diglycerides. In addition, fatty acids such as oleic acid are used in the preparation of injectables. In a particularly preferred embodiment, the cell-targeting complex is suspended in carrier fluid comprising 1% (w/v) sodium carboxymethyl cellulose and 0.1% (v/v) TWEEN 80. The injectable formulations can be sterilized, for example, by filtration through a bacteria-retaining filter, or by incorporating sterilizing agents in the form of sterile solid compositions which can be dissolved or dispersed in sterile water or other sterile injectable medium prior to use.

Compositions for rectal or vaginal administration are preferably suppositories which can be prepared by mixing the cell-targeting complex with suitable non-irritating excipients or carriers such as cocoa butter, polyethylene glycol, or a suppository wax which are solid at ambient temperature but liquid at body temperature and therefore melt in the rectum or vaginal cavity and release the cell-targeting complex.

Dosage forms for topical or transdermal administration of an inventive pharmaceutical composition include ointments, pastes, creams, lotions, gels, powders, solutions, sprays, inhalants, or patches. The cell-targeting complex is admixed under sterile conditions with a pharmaceutically acceptable carrier and any needed preservatives or buffers as may be required. Ophthalmic formulations, ear drops and eye drops are also contemplated as being within the scope of this invention. The ointments, pastes, creams and gels may contain, in addition to the cell-targeting complex of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof. Transdermal patches have the added advantage of providing controlled delivery of a compound to the body.

Such dosage forms can be made by dissolving or dispensing the cell-targeting complex in a proper medium. Absorption enhancers can also be used to increase the flux of the compound across the skin. The rate can be controlled by either providing a rate controlling membrane or by dispersing the cell-targeting complex in a polymer matrix or gel.

Powders and sprays can contain, in addition to the cell-targeting complex of this invention, excipients such as lactose, talc, silicic acid, aluminum hydroxide, calcium silicates and polyamide powder, or mixtures of these drugs. Sprays can additionally contain customary propellants such as chlorofluorohydrocarbons.

When administered orally, the cell-targeting complex are preferably, but not necessarily, encapsulated. A variety of suitable encapsulation systems are known in the art ("Microcapsules and Nanoparticles in Medicine and Pharmacy, "Edited by Doubrow, M.., CRC Press, Boca Raton, 1992; Mathiowitz and LangerJ. Control. Release 5: 13,1987 ; Mathiowitz et al., ReactivePolypers 6: 275,1987 ; Mathiowitz et al. , J Appl. Polymer Sci. 35: 755,1988 ; LangerAcc. Chem. Res. 33: 94,2000 ; Langer J Control. Release 62: 7,1999 ; Uhrich et al., Chem. Rev. 99: 3181,1999 ; Zhou et al.,J. Control. Release 75: 27,2001 ; and Hanes et al., Pharm. Biotechnol. 6:389, 1995).

The cell-targeting complex may be encapsulated within biodegradable polymeric microspheres or liposomes. Examples of natural and synthetic polymers useful in the preparation of biodegradable microspheres include carbohydrates such as alginate, cellulose, polyhydroxyalkanoates, polyamides, polyphosphazenes, polypropylfumarates, polyethers, polyacetals, polycyanoacrylates, biodegradable polyurethanes, polycarbonates, polyanhydrides, polyhydroxyacids, poly (ortho esters) and other biodegradable polyesters. Examples of lipids useful in liposome production include phosphatidyl compounds, such as phosphatidylglycerol, phosphatidylcholine, phosphatidylserine, phosphatidylethanolamine,sphingolipids, cerebrosides and gangliosides.

Pharmaceutical compositions for oral administration can be liquid or solid.

Liquid dosage forms suitable for oral administration of inventive compositions include pharmaceutically acceptable emulsions, microemulsions, solutions, suspensions, syrups and elixirs. In addition to an encapsulated or unencapsulated cell-targeting complex, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof. Besides inert diluents, the oral compositions can also include adjuvants, wetting agents, emulsifying and suspending agents, sweetening, flavoring and perfuming agents. As used herein, the term "adjuvant" refers to any compound which is a nonspecific modulator of the immune response. In certain preferred embodiments, the adjuvant stimulates the immune response. Any adjuvant may be used in accordance with the present invention. A large number of adjuvant compounds is known in the art (Allison, Dev. Biol. Stand. 92: 3,1998 ; Unkeless et al.,Annu. Rev. Immunol. 6: 251,1998 ; and Phillips et al., Vaccine 10: 151,1992).

Solid dosage forms for oral administration include capsules, tablets, pills, powders and granules. In such solid dosage forms, the encapsulated or unencapsulated cell-targeting complex is mixed with at least one inert, pharmaceutically acceptable excipient or carrier such as sodium citrate or calcium phosphate and/or (a) fillers or extenders such as starches, lactose, sucrose, glucose, mannitol and silicic acid, (b) binders such as, for example, carboxymethylcellulose, alginates, gelatin, polyvinylpyrrolidinone, sucrose and acacia, (c) humectants such as glycerol, (d) disintegrating agents such as agar-agar, calcium carbonate, potato or tapioca starch, alginic acid, certain silicates and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay and (i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills and granules can be prepared with coatings and shells such as enteric coatings and other coatings well known in the pharmaceutical formulating art.

It will be appreciated that the exact dosage of the cell-targeting complex is chosen by the individual physician in view of the subject to be treated. In general, dosage and administration are adjusted to provide an effective amount of the cell-targeting complex to the subject being treated. As used herein, the "effective amount" of an cell-targeting complex refers to the amount necessary to elicit the desired biological response. As will be appreciated by those of ordinary skill in this art, the effective amount of cell-targeting complex may vary depending on such factors as the desired biological endpoint, the drug to be delivered, the target tissue, the route of administration, etc. For example, the effective amount of cell-targeting complex containing an anti-cancer drug might be the amount that results in a reduction in tumor size by a desired amount over a desired period of time. Additional factors which may be taken into account include the severity of the disease state; age, weight and gender of the subject being treated; diet, time and frequency of administration; drug combinations; reaction sensitivities; and tolerance/response to therapy. Long acting pharmaceutical compositions might be administered every 3 to 4 days, every week, or once every two weeks depending on half-life and clearance rate of the particular composition.

The cell-targeting complex of the invention is preferably formulated in dosage unit form for ease of administration and uniformity of dosage. The expression "dosage unit form" as used herein refers to a physically discrete unit of cell-targeting complex appropriate for the subject to be treated. It will be understood, however, that the total daily usage of the compositions of the present invention will be decided by the attending physician within the scope of sound medical judgment. For any cell-targeting complex, the therapeutically effective dose can be estimated initially either in cell culture assays or in animal models, usually mice, rabbits, dogs, or pigs. The animal model is also used to achieve a desirable concentration range and route of administration. Such information can then be used to determine useful doses and routes for administration in humans.

Therapeutic efficacy and toxicity of cell-targeting complex can be determined by standard pharmaceutical procedures in cell cultures or experimental animals, e.g. , ED50 (the dose is therapeutically effective in 50% of the population) and LD50 (the dose is lethal to 50% of the population). The dose ratio of toxic to therapeutic effects is the therapeutic index and it can be expressed as the ratio, LD50/ED50. Pharmaceutical compositions which exhibit large therapeutic indices are preferred. The data obtained from cell culture assays and animal studies are used in formulating a range of dosage for human use.

The invention will now be illustrated by way of the following, non-limiting examples.

These examples illustrate the possibilities of the present invention to synthesise cell-targeting complexes with different classes of drugs and different classes of targeting moieties. Furthermore, they illustrate the possibilities of the present invention to developed constructs aimed at different target cells in different organs and diseases. Thus, it can be shown that the novel linker technology is not restricted to a single drug or class of drugs, nor to a single type of targeting moiety nor a single target cell type.

Rather, the cell-targeting complexes of the present invention may for instance target cells that play a major role in liver fibrosis (activated hepatic stellate cells), in renal fibrosis (kidney proximal tubular cells), in chronic inflammatory disorders (activated endothelial cells) or tumour angiogenesis (angiogenic endothelial cells). The drugs incorporated in the complexes may for instance intervene in inflammatory or fibrotic processes (pentoxyfillin, kinase inhibitors, angiotensin II receptor blockers, pyrrolidine dithiocarbamate), or programmed cell death (TNF related apoptosis inducing ligand). The potential usefulness of the synthesised cell targeting complexes will be demonstrated in drug targeting experiments with in vitro cultured target cells and in drug targeting studies in animal models.

### EXAMPLES

### 1. Synthesis of cell targeting complexes with Pt(en(NO3)Cl linker.

A general protocol was developed for the synthesis of drug-carrier conjugates with the Pt(en(NO3)Cl) linker, which will be named cisULS linker hereafter. Typically, this type of cell targeting complexes was prepared by conjugation of the drug to the platinum containing linker, followed by reaction of the linker to a macromolecular carrier. In some of the examples, this approach yields a drug targeting preparation after the second reaction step. In another example, the complex was further modified with homing ligands to yield the final cell targeting complexes.

### 1.a. Synthesis of PTX-cisULS-M6PHSA

In the present example, the anti-inflammatory compound pentoxifyllin (PTX) will be conjugated to the carrier protein mannose-6-phosphate modified human serum albumin (M6PHSA). M6PHSA binds to the insulin-like growth factor II/mannose-6-phosphate receptor, which is highly expressed during liver fibrosis on activated hepatic stellate cells (HSC)[1]. Typically, platinum(ethylenediamine) dichloride (Pt(en)C12; 82.5 mg, 0.253 mmol) was dissolved in 3 ml of dimethylformamide (DMF) and converted into the more reactive Pt(en(NO3)Cl (cisULS mononitrate) by adding small aliquots of AgNO3 (38.5 mg, 0.227 mmol, dissolved in 1 ml of DMF) within a time period of two hours. The precipitated silver chloride was removed by centrifugation. PTX (43.2 mg, 0.155 mmol, dissolved in 1 ml of DMF) was added and the resulting mixture was stirred at 60°C for 2 days. DMF was removed by rotary evaporation, after which the residue was dissolved in 2 ml of water and again taken to dryness under reduced pressure. The crude product was dissolved in water (2 ml) and stored overnight at -20°C, after which unreacted Pt(en)C12 precipitated as yellow crystals, which were removed by centrifugation (10 minutes at 14000 rpm). The identity and purity of the final product PTX-cisULS was checked by HPLC (95%), NMR and mass spectroscopy. PTX-cisULS was stored at 4°C until further use.
Yield: 105 mg (0,166 mmol, 107% yield).
PTX-cisULS ¹H NMR (D₂O): δ_{H} 1.62 (m, 4H, CH₂(C**H₂)₂**CH₂), 2.25 (s, 3H, C**H₃**CO), 2.58 (m, 6H, COC**H₂**, H2N(C**H₂)₂**NH₂), 3.99 (t, J = 6.56 Hz, 2H, C**H₂**N), 4.02 (s, 3H, CONC**H₃**), 4.55 (s, 3H, CNC**H₃**C), 5.59 (m, 4H, **H₂**N(CH₂)₂N**H₂**), 8.48 (s, 1H, C**H**) ppm.
PTX-cisULS ¹⁹⁵Pt NMR (D₂O): δPt -2471 ppm.
PTX-cisULS ionspray MS: The observed product peaks corresponded to PTX-ULS monochloride (MW = 568.1 Da) and to the PTX-ULS compound in which chloride was replaced by a hydroxyl anion (MW = 550.1 Da), which is possibly formed during dilution or ionisation of the compound for mass analysis. Both PTX-ULS species will react equally to the carrier in the second reaction step. M6PHSA was synthesised as described previously [2] and stored after lyophilisation at -20°C until further use. To conjugate PTX-cisULS to M6PHSA, 10 mg of M6PHSA (14.3 nmol) was dissolved in 1 ml of 20 mM tricine/NaNO3 buffer pH 8.3. PTX-cisULS (143 nmol) was added and the pH was checked and corrected if necessary. The mixture was reacted overnight at 37°C, after which unreacted PTX-cisULS was removed by size-exclusion chromatography using disposable PD10 columns (Amersham Biosciences), or by dialysis against PBS at 4°C. The final product was sterilised by filtration via a 0.2 µm filter and stored at -20°C.

PTX-cisULS-M6PHSA was characterised for protein content (BCA method) and for drug content by HPLC analysis. Typically, the coupled drug was released from M6PHSA by overnight incubation with 0.5M KSCN in PBS at 80°C. The released PTX was determined using a standard HPLC system equipped with a UV detector operated at 274 nm and a thermostated column oven operated at 40°C. Elutions were performed on a µBondapak Guard-pak C18 precolumn in combination with a 5 µm Hypersil BDS C8 column (250x4.6 mm, Thermoquest Runcorn, UK) using a mobile phase consisting of acetonitrile/water/trifluoracetic acid (25/75/0.1) at 1 ml/min. PTX eluted at 6 min after injection. Typically, the product contained 5-8 coupled drug molecules per M6PHSA when a 10-fold molar excess was used in the reaction.

### 1.b. Synthesis of losartan-cisULS-M6PHSA

In the present example, the angiotensin II receptor antagonist losartan will be conjugated to the carrier protein M6PHSA. As described above, this carrier binds to activated hepatic stellate cells (HSC) within the fibrotic liver. The coupled drug losartan interferes in the renin-angiotensin system.

To prepare losartan-cisULS, cis-[Pt(ethylenediamine)nitrate-chloride] was formed from platinum(ethylenediamine) dichloride using the general procedure with AgNO3 as described above. 1.5 ml of the resulting cis-[Pt(ethylenediamine)nitrate-chloride] solution (32 µmol) was added to 1.5 ml of a solution of losartan (32 µmol, 10 mg/ml of the potassium salt of losartan in DMF). The resulting solution was heated at 60°C for 3 days during which consumption of the starting material was monitored by analytical HPLC. Mass spectrometry analysis confirmed the presence of the 1:1 losartan-cisULS species after completion of the reaction.
¹H NMR of Losartan-cisULS (CD₃OD): δ_{H} 0.79 (m, 3H, CH₃), 1.25 (m, 2H, C**H₂**CH₃), 1.48 (m, 2H, C**H₂**CH₂CH₃), 2.50 (m, 6H, CH₂C**H₂**C and C**H₂**NH₂), 4.43 (m, 1.8H, NCH₂C), 5.18 (m, 2.2H, C**H₂**OH and remaining NCH₂C), 5.42 (m, 4H, NH₂); cyclic Hs: 6.82 (m, 0.2 H), 6.87 (m, 1.8 H), 7.04 (t, J = 8.06 Hz, 1H, CHCHCH), 7.18 (m, 0.5H), 7.28 (m, 1H), 7.38 (m, 3H), 7.88 (m, 0.5 H). ¹⁹⁵Pt NMR of Losartan-cisULS (CD₃OD): δ_{Pt} -2491 and -2658 ppm.
MS (ESI⁺) m/z: 695 [M-K⁺-OH⁻-H]⁺, 677 [M-K⁺-Cl⁻-H⁺]⁺, 659 [M-K⁺-Cl⁻-H⁺-Cl-+OH⁻]⁺.

Losartan-cisULS was conjugated according to a similar protocol as described for PTX-cisULS conjugation to M6PHSA. In brief, 10 mg of M6PHSA (14.3 nmol) was dissolved in 1 ml of 20 mM tricine/NaNO3 buffer pH 8.3. Losartan-cisULS (143 nmol) was added in a 10-fold molar excess and the pH was checked and corrected if necessary. The mixture was reacted overnight at 37°C, after which unreacted PTX-cisULS was removed by dialysis against PBS at 4°C. The final product was sterilised by filtration via a 0.2 µm filter and stored at -20°C. Losartan-cisULS-M6PHSA contained approximately 7 coupled losartan molecules per mole of protein, as was calculated form its protein content and drug content. These parameters were determined via similar methods as described for PTX-cisULS-M6PHSA. HPLC analysis of losartan was carried out using a Novapak C18 column and a mobile phase consisting of acetonitrile/water/trifluoroacetic acid at a 23/77/0.1 ratio. Eluted compounds were monitored at 225 nm.

### 1.c. Synthesis of SB202190-cisULS-RGDPEG-HSA

In the present example, the p38 MAPkinase inhibitor SB202190 will be conjugated to the carrier protein human serum albumin (HSA). The resulting product will be equipped with RGDpeptide homing ligands that bind to αᵥβ₃ integrin, a receptor that is expressed by endothelial cells in newly formed blood vessels [3]. Furthermore, the HSA core protein will be equipped with polyethylenglycol (PEG) groups, which infer stealth properties in the final construct. As such, these PEG ligands can prevent the uptake by non-target cell cells macrophages.

In the first reaction step, cis-[Pt(ethylenediamine)nitrate-chloride] was synthesised by reacting cis-[Pt(ethylenediamine)dichloride] in *N,N'-*dimethylformamide (5 mg/ml) with one molar equivalent of A_{g}NO₃ (51 mM in N,N-dimethylformamide (DMF). The precipitated silver chloride was removed by centrifugation. Meanwhile, SB202190 was dissolved in DMF at a concentration of 10 mg/ml. Then 440 µl of the cis-[Pt(ethylenediamine)nitrate-chloride] solution (5.18 µmol) was added to 180 µl of the SB202190 solution (5.43 µmol). The resulting solution was heated at 37°C for 3 h and the reaction was followed by HPLC. After the reaction was completed, the mixture was evaporated to dryness under reduced pressure, affording a pale yellow solid that was analysed by HPLC, ¹HNMR and electronspray mass spectrometry. The %yield was 92%. Mass Spectrometry analysis confirmed the presence of the target 1:1 drug:cis-[Pt(ethylenediamine)dichloride] species and ¹H NMR studies indicated that binding of SB202190 to cis-[Pt(ethylenediamine)dichloride] takes place via co-ordination of the N- donor of the pyridine ring contained in the drug to the Pt(II) metal.
¹H NMR of SB202190-cisULS (CD₃OD): δ_{H} 2.59 (m, 4H, H₂N(C**H₂**)**₂**NH₂), 5.58 (s, 2H, NH₂), 5.91 (s, 2H, NH₂), 6.89 (d, J = 8.75 Hz, 2H, F(CHC**H)₂**), 7.22 (m, 2H, N(CHC**H)₂**), 7.53 (m, 4H, (C**H**C**H)₂**OH), 7.82 (d, J = 8.73 Hz, 2H,
F(CHC**H)₂**), 8.52 (m, 2H, N(CHC**H)₂**) ppm.
MS (ESI⁺) m/z: 622 [M+H]⁺, 585 [M-Cl⁻-H⁺]⁺.

In the next reaction step, 10 mg of HSA (14.3 nmol) was dissolved in 1 ml of 20 mM tricine/NaNO3 buffer pH 8.5. SB202190-cisULS (143 nmol) was added and the pH was checked and corrected if necessary. The mixture was reacted for 24h at 37°C, after which unreacted SB202190-cisULS was removed by dialysis against PBS at 4°C. The final product was sterilised by filtration via a 0.2 µm filter and stored at -20°C.

SB202190-cisULS-HSA was characterised for protein content (BCA method) and for drug content by HPLC analysis as described below. Furthermore, conjugation of SB202190-cisULS to the protein was verified by taking UV-spectra, since the final product displayed a specific absorption at 334nm which was due to the conjugated drug and not present in the parent HSA.

For HPLC determination of SB202190, the coupled drug was released from HSA by overnight incubation with 0.5M KSCN in PBS at 80°C. The released SB202190 was determined using a standard HPLC system equipped with a C18 µBondapak column and a UV detector operated at 254 nm. Compounds were eluted with a mixture of water/acetonitrile/ trifluoroacetic acid 80/20/0.1 at 1 ml/min. Typically, the product contained 5-8 coupled drug molecules per HSA when a 10-fold molar excess of SB202190-cisULS was used in the reaction.

In the final reaction step, SB202190-cisULS-HSA was modified with PEG and RGD-peptide groups. SB202190-cisULS-HSA dissolved in PBS was treated with a 50-fold molar excess of VNS-PEG-NHS (Nektar, Alabama, USA; 20 mg/ml in water), which was added dropwise. The mixture was protected from light with tin foil and incubated for 1h at room temperature on a rollerbank. Meanwhile, the RGD peptide c(RGDfK-Ata) (Ansynth Service, Roosendaal, The Netherlands) was dissolved at 10 mg/ml in a 1:4 acetonitrile/water mixture. The RGDpeptide was added dropwise to the reaction mixture at a mole:mole ratio of 55:1, after which hydroxylamine was added in a final concentration of 50 mM. The reaction was carried out overnight at room temperature while protected from light. Remaining VNS groups were quenched by addition of cysteine (55:1 molar excess over the amount of HSA), after which the product was dialysed against PBS, and finally purified by size exclusion chromatography using a superdex200 column on an Äkta System (Amersham Pharmacia, Uppsala, Sweden). The final product was stored at -20°C.

SB202190-cisULS-RGDPEG-HSA was characterised for its protein content and drug content as described above for SB202190-cisULS-HSA. Analytical size exclusion chromatography was performed to reveal increase in size, due to PEGylation, and to determine purity of the products. SDS-PAGE followed by western blotting and immunodetection with an in-house prepared anti-RGD-peptide antiserum was performed to demonstrate binding of the RGD peptide to the complex.

### 1.d. Synthesis of SB202190-cisULS-lysozyme

In the present example, the p38 MAPkinase inhibitor SB202190 will be conjugated to the carrier protein lysozyme (LZM). Drug-LZM conjugates are accumulated in the kidney after in vivo administration, since LZM and other low molecular weight proteins undergo glomerular filtration and subsequential uptake in proximal tubular cells [4]. As such, the developed SB202190-cisULS-LZM conjugate can serve in the delivery of the p38 MAPkinase inhibitor to the kidney.

In the first reaction step, SB21290-cisULS was synthesised as described above. Next, the SB202190-cisULS was reacted to the carrier lysozyme. For this purpose, one additional methionine group was introduced into the protein prior to the reacting with the drug-cisULS molecule. In brief, Boc-L-methionine hydroxysuccinimide ester (17 µmol; 10 mg/ml in DMSO) was added to lysozyme (14 µmol; 10 mg/ml in 0.1 M sodium bicarbonate buffer pH 8.5) and stirred for 1 h at room temperature. The product was dialysed against water and lyophilised. Methionine-modified lysozyme (met-LZM) contained one to two extra methionine groups as was confirmed by electron-spray mass analysis. The product was stored at -20°C until further use. Met-LZM was further reacted with SB202190-cisULS according to the following protocol. A solution of met-LZM (2.6 µmol; 10 mg/ml in tricine/sodium nitrate buffer (0.02 M, pH 8.5) was treated with SB202190-ULS (7.8 µmol; 10 mg/ml in DMF), after which the mixture was incubated at 37°C for 24 h. The product was dialysed against water, filtered through 0.2-µm membrane filter, lyophilised and stored at -20°C. The coupling of SB202190 with lysozyme in a 1:1 ratio was confirmed by electron-spray mass analysis of the final product. Furthermore, coupling of the drug was confirmed by HPLC analysis of the coupled SB202190 after release of the drug from the SB202190-lysozyme conjugate. For this purpose, the conjugate was incubated with 0.5M potassium thiocyanate (KSCN) dissolved in 0.1 M phosphate buffer saline at 80°C for 24 h to release SB202190 completely. SB202190 was estimated using HPLC (Waters, Milford, MA, USA) analysis method described elsewhere. Briefly, the samples were added with an internal standard, SB202190 derivative, and extracted with 2 ml of diethylether three times. The extract was dried at 50°C and the residue was reconstituted in 100 µl of mobile phase (acetonitrile:water:trifluroacetic acid, 30:70:0.1 v/v/v). Twenty-five microliter of it was injected into HPLC column (Thermo-Hypersil Keystone, Bellefonte, PA) and detected at 350 nm. The peak height ratios of SB202190 and internal standard were used to calculate the concentration. The degree of SB202190 substitution in the conjugate was found to be 1 mole of SB202190 per mole of lysozyme. No free SB202190 was found in the final preparation.

### 1.e. Synthesis of losartan-cisULS-lysozyme

In the present example, the angiotensin II antagonist losartan will be conjugated to the carrier protein lysozyme (LZM). The resulting product losartan-cisULS-LZM will be accumulated in the kidney after in vivo administration, since LZM and other low molecular weight proteins undergo glomerular filtration and subsequential uptake in proximal tubular cells. As such, the developed conjugate can serve in the delivery of the angiotensin antagonist to the kidney.

Losartan-cisULS was synthesised as described above. Subsequently, this compound was coupled to met-LZM according to a similar protocol as described for SB202190-cisULS conjugation. In brief, losartan-ULS (0.72 µmol; 3 mg/ml in DMF) was added to a solution of met-LZM (0.36 µmol; 10 mg/ml in 20 mM tricine/sodium nitrate buffer pH 8.5) and the mixture was incubated at 37°C for 24 h. The resulting product was purified by dialysis against water for 2 days, filtered through 0.2-µm membrane filter, lyophilised and stored at - 20°C. The coupling of losartan with lysozyme in a 1:1 molar ratio was confirmed by mass analysis of the final product and HPLC analysis of the coupled drug after its release from the carrier according to methods described above.

### 1.f. Synthesis of small drug-trans-[Pt(ethylenediamine)chloride] complexes

The synthesis of *trans*-[Pt(ethylenediamine)dichloride] systems was performed in five steps (see also Figure 1).

### 1.- Cis-[PtCl(ONO₂)(NH₃)₂]

Transplatin (0.1 g, 0.333 mmol) in 4 ml of dimethylformamide (DMF) was treated with 0.9 equiv. of AgNO₃ (0.051 g, 0.3 mmol). The reaction mixture was stirred overnight, in the dark. After this time, AgCl was filtered off and the yellow solution, containing *cis*-[PtCl(ONO₂)(NH₃)₂], was checked by ¹⁹⁵Pt NMR. A single peak was observed at -1775 ppm, which agrees with the ClON2 environment around the platinum centre.

### 2.- Cis-[PtCl(NH₃)₂(Boc1,n)]⁺(n = 3, 4, 5)

*Cis*-[PtCl(ONO₂)(NH₃)₂] in DMF was treated with 0.8 equiv. of Boc1,n (n= 3 (0.046 g, 0.266 mmol), 4 (0.050 g, 0.266 mmol), 5 (0.099 g, 0.266 mmol)) and 0.8 equiv. of triethylamine (Et₃N). The reaction mixture was reacted overnight at room temperature. The formation of *cis-*[PtCl(NH₃)₂(Boc1,n)]⁺ was checked by ¹⁹⁵Pt NMR, directly from the reaction solution. A single signal was observed at -2399 ppm, which was assigned to a ClN3 environment around the platinum. The same signal was observed, at the same chemical shift, for the three used linkers Boc1,3; Boc1,4 and Boc1,5.

### 3.- Cis-[Pt(ONO₂)(NH₃)₂(Boc1,n)]⁺(n = 3, 4, 5)

*Cis*-[Pt(Cl)(NH₃)₂(Boc1,n)]⁺ was treated with 0.9 equiv. of AgNO₃ (0.051 g, 0.3 mmol) in DMF. The reaction mixture was stirred overnight, in the dark. After this time, AgCl was filtered off and the yellow solution, containing *cis*-[Pt(ONO₂)(NH₃)₂(Boc1,n)]⁺, was checked by ¹⁹⁵Pt NMR. A single signal was observed at -2133 ppm. This signal agrees with the ON3 environment around the platinum. The same chemical shift value was observed for the three used linkers.

### 4.-Cis-[Pt(ptx)(NH₃)₂(Boc1,n)]²⁺ (n = 3, 4, 5) where ptx is pentoxyfilline

*Cis*-[Pt(ONO₂)(NH₃)₂(Boc1,n)]⁺ was treated with 0.8 equiv. of pentx (0.074 g, 0.266 mmol) at 75 °C, overnight in the dark. After overnight the formation of the desired species were checked by ¹⁹⁵Pt NMR. A signal was observed at -2487, -2484 and -2492 ppm for complexes RBo1,3, RBoc1,4 and RBoc1,5, respectively. These values are in agreement with a 4N environment around the platinum centre.

### 5.- Deprotection of the Boc group.

The deprotection of the amine group in the final step takes places by the overnight treatment with 0.1 M HCl of each of the complexes RBoc1,3, RBoc1,4 and RBoc1,5 at 50 °C, in the dark. ¹H NMR proved the successful deprotection by the disappearance of the signal assigned to the tert-butyl group at 1.34 ppm.

### Purification of the trans-[Pt(ethylenediamine)chloride] systems by HPLC

Purification via HPLC for all three *trans*-ULS^{®} systems was performed as a mixture of different species was initially formed. Conditions are given in the appropriate section.

For all the complexes the purification was successful, as checked by MS and in ¹H-NMR.

### Modification of the NH₂ group of the linker

For the modification of the amine group, the [Pt(ethylenediamine)chloride] systems have been dissolved in water and the pH is adjusted between 8-9 with 1M NaOH. After the pH is adjusted, 1.5 equiv. of bromoacetic acid N-hydroxysuccinimide ester is added to the solution. The reaction is stirred at room temperature for 7 hr. It is important to maintain the pH of the reaction mixture between 8 and 9 during the complete reaction (Figure 2), to allow the reaction between the amine group and the ester.

The progress of the reaction was followed by LCMS. Every 2 hr an aliquot was withdrawn and checked by LCMS. It was observed that the peak corresponding to the modified complexes was increasing its intensity with time. After 7 hr of reaction no further changes were observed in the chromatogram. The mass of the desired species were found to be 701.08, 715.05, 729.08, respectively. Purification of the modified complexes was required. It was performed by HPLC, with the same conditions as used for the purification of the non-modified complexes and was successful in all cases.

### 1.f. Synthesis and characterisation of dinuclear species:

### Dichloro(ethylenediamine)platinum (II) (1)

Potassium iodide (2g, 12 mmol) was added to a solution of 1 g (2 mmol) of K₂PtCl₄ in 50 ml of water. The resulting dark solution of K₂PtI₄ was treated with 0.16 ml (2.4 mmol) of ethylenediamine and allowed to stand at room temperature for several hours. Then, the dark yellow precipitate of PtenI₂ was collected by filtration, washed with water, ice-cold ethanol and ether, and dried in air. Yield: 1 g (99%).

Silver nitrate (0.66 g, 3.9 mmol) in 5 ml of water was then added to the suspension of PtenI₂ (1 g, 2 mmol) in 25 ml of water upon stirring. The mixture was stirred in the dark overnight at room temperature. Then, the white precipitate of AgI was filtered off, and 0.33 g (4.4 mmol) of KCl was added to the filtrate. The mixture was stored overnight in the dark, and the resulting yellow precipitate was collected by filtration, washed with ice-cold water, ethanol and ether, dried in air. Yield: 0.52 g (80%). Anal. Calcd for C₂H₈N₂Cl₂Pt: C, 7.37; H, 2.47; N, 8.59. Found: C, 7.58; H, 2.80; N, 8.50. ¹⁹⁵Pt NMR (D₂O): δ -2330.

### 2-(2-(tert-butyloxycarbonylamino)ethoxy)ethanol (2a)

A solution of 2.62 g (12 mmol) of di-tert-butyl dicarbonate in 10 ml of ethanol was gently added to a stirred solution of 1.05 g (10 mmol) of 2-(2-aminoethoxy)ethanol in 10 ml of ethanol. After stirring for 4 h at room temperature, the solvent was removed under reduced pressure. The residue was extracted with 25 ml of ethylacetate, and the organic phase was dried (Na₂SO₄), filtered and evaporated to get the product. Yield: 1.95 g (95%). ¹H NMR (CDCl₃): δ 4.88 (s, 1H, NH), 3.74 (t, 2H, H₁), 3.56 (m, 4H, H₂+ H₃), 3.34 (m, 2H, H₄), 1.45 (s, 9H, Boc).

### 1-(2-(tert-butyloxycarbonylamino)ethoxy)-2-sulfonylethane (2b)

Methanesulfonyl chloride (0.88 ml, 11.4 mmol) was gently added to a stirred solution of 1.95 g (9.5 mmol) of **2a** and 2 ml (14.3 mmol) of triethylamine in 50 ml of dichloromethane at 0 °C. The resulting solution was stirred for 1 h at 0 °C and for 30 min at room temperature. Then the solution was successively washed with 1 M HCl (50 ml), water (50 ml), 10% aqueous solution of Na₂CO₃ (50 ml) and brine (50 ml). The organic phase was dried (Na₂SO₄), filtered and evaporated to get the product. Yield: 1.62 g (60%). ¹H NMR (D₂O): δ 4.35 (t, 2H, H₁), 3.73 (t, 2H, H₂), 3.56 (t, 2H, H₃), 3.33 (m, 2H, H₄).

### 1-(2-(tert-butyloxycarbonylamino)ethoxy)-2-azidoethane (2c)

Sodium azide (1.49 g, 22.9 mmol) was added to a stirred solution of **2b** (1.62 g, 5.72 mmol) in 20 ml of anhydrous dimethylformamide. The mixture was heated at 60-70 °C overnight. After cooling, the solution was poured into water and extracted with ethyl acetate (5x30 ml). Organic phases were collected, dried (Na₂SO₄), filtered and evaporated to obtain the product. Yield: 0.6 g (46%). ¹H NMR (CDCl₃): δ 3.65 (t, 2H, H₂), 3.55 (t, 2H, H₃), 3.36 (m, 4H, H₁+ H₄).

### 1-(2-(tert-butyloxycarbonylamino)ethoxy)-2-aminoethane (NONBoc, 2d)

A solution of 0.82 g (3.1 mmol) of triphenylphosphine in 10 ml of tetrahydrofurane was added to a solution of 0.6 g (2.6 mmol) of **2c** in 20 ml of tetrahydrofurane. The resulting solution was stirred for 2 h at room temperature. Subsequently, 200 ml of water was added, and the mixture was stirred overnight at room temperature. Then the mixture was evaporated to 75 ml and filtered. The filtrate was washed with 25 ml of dichloromethane and evaporated to get the product. Yield: 0.5 g (79%). ¹H NMR (CDCl₃): δ 3.50 (m, 4H, H₂+ H₃), 3.33 (m, 2H, H₄), 2.86 (t, 2H, H₁). ESI-MS: m/z 205.2 (M+H).

### [Pt(ethylenediamine)Cl(dmf)](NO₃) (1a)

A solution of 38.6 mg (0.23 mmol) of AgNO₃ in 1 ml of dimethylformamide (DMF) was added portionwise over 1.5 h to a stirred solution of 78.2 mg (0.24 mmol) of **1** in 1.6 ml of DMF at room temperature in the dark. The mixture was stirred for 5 h in the dark, and the AgCl precipitate was then filtered off. The resulting pale-yellow DMF solution of [PtenCl(dmf)](NO₃) was used as a starting material for the preparation of **1b** and **1c** as described below.

### [Pt(ethylenediamine)Cl(NONBoc)](NO₃) (1c)

A solution of **1a** obtained as above was added to 40 mg (0.2 mmol) of NONBoc **(2d)** in the dark. The resulting solution was stirred overnight in the dark at room temperature. Then DMF was removed in vacuo. Excess of water was added to the residue, and a yellow precipitate of PtenCl₂ was filtered off. The remaining filtrate of **1c** was lyophilized. Yield: 87 mg. ¹⁹⁵Pt NMR (H₂O): δ -2645.

### [Pt(ethylenediamine)(ptx)(NONBoc)](NO₃) (1f), route II where ptx is pentoxyfilline

A solution of 19.8 mg (0.117 mmol) of A_{g}NO₃ in 1 ml of DMF was added portionwise over 1.5 h to a stirred solution of 68.6 mg (0.123 mmol) of **1c** in 1.6 ml of DMF at room temperature in the dark. The mixture was stirred for 5 h in the dark, and the AgCl precipitate was then filtered off. The filtrate le was added to 27.4 mg (0.098 mmol) of ptx. The resulting solution was stirred in the dark at 70 °C. Then it was filtered off the black precipitate, and evaporated. The residue was dissolved in water, filtered, and the filtrate of 1f was lyophilized. Yield (crude product): 71 mg. ¹⁹⁵Pt NMR (DMF): δ -2700. ESI-MS: m/z 737.2 (M―2(NO₃)―H), 459.28 (M―2(NO₃) ―PTX ―H).

### [{Pt(ethylenediamine)(ptx)}(µ-NON){Pt(ethylenediamine)X}](CH₃COO)₃, X = Cl (4) or CH₃COO (4a)

Complex **1f** (81.4 mg, 0.094 mmol) was dissolved in 4 ml of 0.1M hydrochloric acid, and the resulting solution was heated overnight in the dark at 50 °C. After that, pH of the solution was adjusted to 8 with 2M NaOH. Then 68 mg (0.4 mmol) of silver nitrate was added, in order to remove chloride ions from the solution. The precipitate of AgCl was filtered off, and subsequently a solution of **1a** obtained as described above was added to the filtrate. The resulting solution was stirred at 50 °C for 24 h in the dark. The solvent was then removed in vacuo. The residue was dissolved in water, filtered and purified by high performance liquid chromatography as described above. The purified product was lyophilized and characterized by ¹H and ¹⁹⁵Pt NMR and LC ESI-MS. Yield: 5 mg. ¹H NMR (D₂O): δ 8.61 (s, 1H, ptx), 4.50 (s, 3H, ptx), 4.09 (s, 3H, ptx), 4.01 (t, 2H, NON), 3.72 (t, 2H, NON), 3.64 (m, 4H, NON), 2.81 (m, 2H, en), 2.78 (m, 2H, en), 2.65 (m, 4H, ptx), 1.93 (s, CH₃COO), 1.62 (m, 4H, ptx). ¹⁹⁵Pt NMR (D₂O): δ -2400 **(4a), -** 2632 **(4),** -2700. LC ESI-MS: retention time for **4** 9.68 min (m/z 985.14 (M(**4**)³⁺-2H+CH₃COO), 647.04 (M(**4**)³⁺-3H-ptx); retention time for **4a** 12.09 min (m/z 1009.08 (M(**4a**)³⁺-2H +CH₃COO), 669.93 (M(**4a**)³⁺-3H-ptx).

### 1.g. Synthesis and characterisation of heterobifunctional linkers: SMCC-[Pt(ethylenediamine)chloride]

20.2 mg of KRE-Boc-ULS™ (batch#81620000; 3.54*10⁻⁵ mol) were dissolved in 577.14 µL of a 200 mM solution of hydrochloric acid in MilliQ. The resulting solution was heated at 50oC overnight. 264.52 µL of 200 mM hydrochloric acid in MilliQ then were added. The pH was adjusted to about 7.5 using a 1 M solution of sodium hydroxide in MilliQ and a 200 mM solution of hydrochloric acid in MilliQ. The pH of a 0.05 M phosphate buffer was adjusted to 7.25 using a 1 M solution of sodium hydroxide in MilliQ. 561.11 µL of the resulting buffer solution then were added to the solution of deprotected KRE-Boc-ULS™. 11.9 mg (3.56*10⁻⁵ mol) of the SMCC succinimidyl ester were dissolved in about 5 drops of *N,N'*-dimethylformamide and added dropwise to the ULS™ solution. Precipitation appeared to occur. Therefore, was added dropwise until solubility was achieved. The resulting solution was stirred overnight at ambient temperature under protection from light. Mass Spectrometry analysis then was performed.

### 2. In vitro evaluation of cell targeting complexes

### 2.a. Drug release with PTX-cisULS-M6PHSA

The stability of the drug-carrier linkage was investigated by incubating PTX-cisULS-M6PHSA at 37°C under several conditions. First, the stability of the conjugate in biological media was investigated by incubating the PTX-cisULS-M6PHSA conjugate up to 7 days in a 1:1 dilution of normal rat serum and PBS. Release of PTX from the conjugate was assessed by treating aliquots with acetonitrile (2:1 v/v) after which the samples were centrifuged at high speed to precipitate proteins. The clear supernatant was stored at -20°C until HPLC analysis.

Second, to test whether thiol containing compounds can displace PTX from the conjugate, drug release studies were performed according to the same protocol by incubating the PTX-cisULS-M6PHSA conjugate for 24h at 37°C in PBS or in PBS containing 5 mM glutathione (GSH) or 5 mM dithiothreitol (DTT).

### Results

The drug release profiles of PTX-M6PHSA conjugate are depicted in Figure 4. The released drug was related to the total amount of coupled PTX As is shown in panel A of this figure, PTX-cisULS-M6PHSA only slowly released drug in serum indicating that the linkage between drug and carrier displays adequate stability which enables the conjugate to reach the target cells. Alternatively, the observed release profile would allow for a slow release profile of the drug from a drug carrier that is not actively binding to specific target cells. Such a product could liberate the drug continuously, thereby controlling drug levels within the body. Examples of such preparation are for instance a subcutaneously injected depot preparation or a carrier that circulates in the blood stream.

Furthermore, the results of the second experiment, as shown in panel B of Fig 4, indicate that the linkage between drug and carrier displays adequate release characteristics in an environment that resembles intracellular conditions. Thus, the parent drug PTX can, in principle, be generated from the conjugate inside target cells.

### 2.b. In vitro effects of PTX-cisULS-M6PHSA conjugates on HSC

The pharmacological effects of the PTX-cisULS-M6PHSA conjugates were tested on activated HSC. HSC were isolated from livers of male Wistar rats (0.3kg) according to standard techniques [5] and cultured in disposable plastic bottles (Nunc, USA), in DMEM (GIBCO) containing 10% fetal calf serum (Hyclone Laboratories Inc., Logan, UT, USA), 100 U/ml penicillin, and 100µg/ml streptomycin. These culture conditions allow HSC to attach and proliferate as confirmed by microscopic evaluation. Cells cultured for 2 days after the isolation exhibited the phenotype of quiescent HSC, a compact cell shape with many cytoplasmic fat droplets, while cells cultured for 10 days after isolation displayed the more elongated phenotype of activated HSC without the vitamin A containing fat droplets. All experiments with conjugates were performed with the latter type of activated HSC (HSC day 10). Typically, HSC were incubated for 24h at 37°C with the conjugates. Subsequently, the cells were subjected to analyses related to antifibrotic effects of the coupled PTX or to cytotoxicity of the platinum linker, immunostaining of fibrosis markers or the caspase 3/7 activity assay, respectively.

### Antifibrotic effects of conjugates

HSC (10.000 cells/well in parmenox LAB-TEK chamber slides) were incubated with culture medium spiked with either PTX-cisULS-M6PHSA, PTX-cisULS-HSA, or FLU-ULS-M6PHSA, all at a concentration of 1 mg/ml, or spiked with 1 mM PTX. Assuming a 6:1 coupling ratio, this concentration of PTX-cisULS-M6PHSA corresponds to 100 µM of coupled PTX. After 24h of incubation, HSC were acetone-fixed and stained for the presence of fibrosis markers according to standard methods using monoclonal antibodies against a-smooth muscle actin (Sigma) or collagen type I (Southern Biotechnology, USA). HSC morphology was analysed via direct optical microscopy observation.

### Caspase 3/7 activity assay

HSC (10.000 cells/well seeded in white-walled 96 well-plates, Corning Costar) were incubated in culture medium spiked with either cisplatin (100 µM), PTX-cisULS (100 µM), PTX-cisULS-M6PHSA (1 mg/ml), or fluorescein-ULS-M6PHSA (1 mg/ml). Assuming a coupling ratio of 6:1, these latter protein concentrations correspond to an equivalent platinum concentration of 100 µM. After 24h, induction of apoptosis was determined by caspase 3/7 activity measurement using the apoptosis Caspase-Glo assay (Promega).

### Results

One of the hallmarks of liver fibrosis is the induced expression and production of a-smooth muscle actin (aSMA) and collagen type 1 by activated HSC. These proteins were therefore selected as read-out parameters of the antifibrotic effects of the targeted PTX. As can be seen in Figure 5, activated HSC express collagen type I in a granular staining pattern in the cytoplasm, probably corresponding to the presence of procollagen type I, while aSMA stained in a fiber-like pattern (panel A,B). Treatment with PTX in a concentration of 1 mM reduced the intensity of both collagen type I production and aSMA (panel G,H). Incubation of the cells with 1 mg/ml PTX-cisULS-M6PHSA, which corresponds to 100 µM conjugated PTX, affected the collagen type I expression after 24h of incubation considerably, as can be observed in the reduced intensity of the staining (panel E). Although the aSMA-staining intensity was not affected, pronounced changes in the morphology of the stellate cells were observed after the incubation with PTX-cisULSM6PHSA. Especially after staining of the abundantly present aSMA, the changes in the structure of the cells became clearly visible (panel F). HSC reacted to the treatment of PTX-cisULS-M6PHSA with a loss of cytoplasmic volume, rounded cell shape and detachment of the cells from the surface of the plates. These effects were not observed after incubation with equivalent concentrations of PTX-HSA (panel C,D) or fluorescein-ULS-M6PHSA (data not shown), indicating that the effect was induced by the drug PTX targeted by the carrier M6PHSA.

Since cisplatin is a well-known antitumor agent, the use of platinum-coordination chemistry for linking drugs to a carrier may infer unwanted toxicity into the construct. The effects of the conjugates on cell viability and apoptosis were therefore evaluated. Treatment of activated HSC with cisplatin for 24 h induced apoptosis of this cell type, as reflected in activation of caspases 3 and 7 (Figure 6). In contrast, PTX-cisULS and the conjugates prepared with PTX-cisULS (PTX-cisULS-M6PHSA and PTX-cisULS-HSA) did not induce activation of apoptotic events in the caspase assay. From this, we conclude that the PTX-cisULS-M6PHSA conjugate is not displaying cisplatin-like effects in HSC.

### 2.c. In vitro effects of SB202190-cisULS-RGDPEG-HSA on TNfalfa-activated HUVEC

The effect of SB202190-cisULS-RGDPEG-HSA has been studied on human umbilical cord endothelial cells (HUVEC) cultured in vitro. In brief, HUVEC were incubated for 48h with SB202190, RGDPEG-HSA and SB202190-cisULS-RGDPEG-HSA. Tumor necrosis factor alpha (TNF) at a final concentration of 10ng/ml was added 24h after start of incubation as an inducer of inflammatory signalling events. Cells and culture medium was harvested 24h later and used for quantitative real time RT-PCR detection of inflammation-related genes or ELISA-based detection of secreted IL-8.

### Results

Figure 7, panel A shows the gene expression levels of IL8 in HUVEC treated with TNF and the described compounds. As can be appreciated, the IL8 gene is upregulated in HUVEC 8 as response to treatment with TNF. This response can be inhibited with SB202190 at concentration of 10 µM to a level of 40%. Treatment with SB202190-cisULS-RGDPEG-HSA results in inhibition of this gene although to a lesser extent. Treatment with RGD-PEG-HSA did not reduce IL8 gene expression.

Furthermore, we demonstrated that inhibition of IL-8 gene expression also corresponded to a reduced production and secretion of IL8 in the culture medium (Fig 7 panel B).

From these two results we conclude that the p38 MAPkinase inhibitor SB202190 can be generated from the cell targeting complex once the compound is processed by the cells. The observed difference in potency of the drug can be explained by the cellular routing of the conjugate, i.e. the drug targeting construct has to be internalized and processed in the lysosomal compartments of the cell to release the active drug. These processes take longer than the simple passive diffusion of the free compound. Combined with the different behaviour of the carrier in vivo however, the RGD-targeted drug may demonstrate a superior activity versus the free compound.

### 2.d. In vitro cytotoxic effects of drug-cisULS-lysozyme conjugates on kidney tubular cells

Since nephrotoxicity is one of the major side effects associated with cisplatin treatment, we carefully examined the safety of the cell targeting complexes directed to the kidney. For this purpose, we incubated drug-cisULS products and drug-cisULS-lysozyme conjugates with kidney proximal tubular cells and assayed the cell-viability after 24h. In brief, NRK-52E rat proximal tubular cells were seeded at 10⁴ cells/well in 96-well plates in normal growth medium (DMEM + 5% FCS + 4mM L-glutamine + penicillin-streptomycin). After 24 h of culture, the medium was replaced with the medium containing different compounds and cells were grown further for 24 h. Cell viability was determined by incubating the cells for 4 h with 20 µl of AlamarBlue (Serotec Ltd., Oxford, UK), followed by fluorimetric detection of the formed product. Cell viability of control cells was set at 100%.

### Results:

Upon 24h of incubation with cisplatin, a clear reduction in cell viability was observed (Table 1). In contrast, all other treatments did not show a platinum-related effect on cell viability. As can be observed in table 1, treatment with drug-cisULS compounds affected the cell viability similar as treatment with the parent drugs conjugated to the platinum linker (group 1) . Similarly, SB202190-cisULS-LZM did not affect cell viability other than the effect observed after incubation with non-modified lysozyme (group 2). From this result, we conclude that the platinum linker is not displaying platinum-related toxicity when incubated with renal tubular cells.

**Table 1. Effect of cis-ULS-conjugated compounds on viability of kidney tubular cells.**

| | |
|---|---|
| To evaluate the (absence of) platinum-related toxicity of the cis-ULS linker when incorporated in cell targeting complexes, rat tubular epithelial cells (NRK52E cells) were incubated with cisplatin (positive control, group 1), drug-cisULS adducts or parent drug (group 2), cell targeting complexes or parent carrier (drug-cisULS-LZM conjugates, group 3). Cell viability was determined using the Alamar Blue assay. (see description for more details) | |

| **Samples** | **% cell viability (mean ± SEM)** |
|---|---|
| Medium | 100 |
| Cisplatin (100µM) | 24.6** ± 7.0 **p<0.01 |

| Group-1: drug-cisULS compounds | |
|---|---|
| SB202190 (100µM) | 68.5 ± 10.7 |
| SB202190-cisULS (100µM) | 62.7± 3.7 |
| Losartan (100 µM) | 76.4 ± 10.0 |
| Losartan-cisULS (100µM) | 78.6 ± 6.4 |

| Group-2: Lysozyme conjugates | |
|---|---|
| SB202190-cisULS-LZM (equivalent to 100µM platinum) | 70.4 ± 7.4 |
| Lysozyme (equivalent to the amount of SB202190-cisULS-LZM) | 70.7 ± 5.4 |

### 3. In vivo evaluation of cell targeting complexes

### 3.a. Pharmacokinetic evaluation of SB202190-cisULS-LZM in rats.

In order to demonstrate the potential applicability of the newly developed cell targeting complex, we administered SB202190-cisULS-LZM to healthy rats and determined the concentrations of the drug in the serum and organs of the animals. Since the SB202190-cisULS-LZM is supposed to accumulate in the kidney, renal concentrations of the drug indicate whether drug targeting has been achieved successfully. Knowledge on other drug-lysozyme conjugates that have been investigated in the past enables us to interpret the drug profiles.

All animal experiments were approved by Animal Ethics Committee of the University of Groningen. Upon arrival, male Wistar rats (Harlan, Zeist, The Netherlands, 220-240 g) were housed in a temperature-controlled room with a 12 hour light/dark cycle and offered *ad libitum* intake of tap water and standard rat chow (HopeFarm Inc., Woerden, The Netherlands). Rats were injected intravenously with a single dose of the SB202190-cisULS-lysozyme conjugate (16 mg/kg equivalent to 376 µg/kg of SB202190; dissolved in 5% glucose) via the penile vein. Animals were sacrificed at different time points between 1 and 72h after administration. Blood samples were collected by heart puncture and kidneys were isolated after flushing the organs gently with saline. Blood samples were kept on ice for 1 h and then serum was separated by centrifuging the samples at 3000g for 10 min. Serum and organs were snap-frozen into liquid nitrogen immediately. Urine samples were collected using metabolic cages and combined with the urine collected from urinary bladder after sacrificing the animals. Kidneys were weighed, homogenized (1:3 w/v) in 0.1M ice-cold phosphate buffer saline (pH 7.5) using an Ultra Turrax T25 homogenizer (IKA, Stauffen, Germany) and then stored at -80°C. Free SB202190 levels in serum, kidneys and urine were estimated by HPLC analysis as described above. To estimate the total SB202190 levels (free SB202190 plus SB202190 coupled to the linker), 100 µl of the samples was incubated for 24 h with KSCN (0.5 M dissolved in PBS) at 80°C to release SB202190 completely. Then the samples were further processed to measure SB202190 by HPLC as described above.

In addition to the SB202190 analysis by HPLC, we determined whether the SB202190-cisULS-LZM had accumulated in the proximal tubular cells of the kidney by immunostaining with an anti-lysozyme antibody. For this purpose, cryostat section of kidneys were analysed by standard immunohistochemical techniques.

### Results:

The renal levels of both total SB202190 (the sum of drug still boudn to the linker and released drug) and released SB202190 are shown in Table 2. As can be appreciated from this table, administration of a single dose of the SB202190-cisULS-LZM conjugate resulted in sustained renal levels of the delivered drug. Since the drug can, in principle, exert its action only upon liberation from the conjugate, the observed levels of total drug can be considered as a reservoir of locally present drug that generates sustained levels of free drug within the kidney. In contrast, no free SB202190 could be detected in the serum.

Next to the drug analysis, we demonstrated the presence of the SB202190-cisULS-LZM conjugate using anti-lysozyme immundetection (fig 8). Arrows indicate the presence of the conjugate in proximal tubular cells, which is the intended target cell of the lysozyme carrier.

Another striking result can be appreciated when the amount of drug that accumulates in the kidneys is compared with the distribution of non-targeted SB202190. Upon administration of a much higher dose, 5 mg/kg of free drug versus 400 ug/kg of the targeted drug, only 0.2% distributed to the kidney, resulting in a concentration of 0.9 ug/g at sacrification of the animals at 4h after administration. In contrast, the SB202190-cisULS-LZM conjugate accumulated for about 20% to the kidneys, a value that is comparable to the values observed with other drug-lysozyme conjugates. Furthermore, the conjugate resulted in persistent levels of free SB202190 up to 3 days after administration, while only transient levels of SB202190 can be expected after administration of free drug. From these results, we conclude that the SB202190-lysozyme conjugate prepared with the cisULS linker shows a promising drug-release profile, enabling sustained release of the conjugated from within the target cells. These characteristics make it an excellent conjugate for drug targeting purposes.

**Table 2. Pharmacokinetic data of SB202190-cisULS-LZM.**

| | | | |
|---|---|---|---|
| Male Wistar rats were injected intravenously with either SB202190-cisULS-LZM or with non-targeted SB202190. At indicated time points, the animals were sacrificed and free and total drug concentrations were determined in the homogenized kidneys as described herein. Note the difference in the administered doses of targeted and non-targeted drug. (See description for more details) | | | |

| **drug** | **dose** | **total renal SB202190 levels (%dose)** | **free renal SB202190 levels (µg/g)** |
|---|---|---|---|
| SB202190-cisULS-LZM | 16 mg/kg conjugate corresponding to 0.4 mg/kg SB202190 | 19.9% (at 1h) | 0.6 (at 1h) |
| | | 21.7% (at 6 h) | 1.0 (at 6 h) |
| | | 18.1% (at 12 h) | 1.1 (at 12 h) |
| | | 21.5% (at 24 h) | 2.0 (at 24 h) |
| | | 16.5% (at 48h) | 0.5 (at 48h) |
| | | 11.9% (at 72h) | 0.5 (at 72h) |
| Free SB202190 | 5.0 mg/kg | 0.2% (at 4h) | 0.8 (at 4h) |

### REFERENCES

[1] L. Beljaars, G. Molema, B. Weert, H. Bonnema, P. Olinga, G.M.M. Groothuis, D.K.F. Meijer, and K. Poelstra, Albumin modified with mannose 6-phosphate: A potential carrier for selective delivery of antifibrotic drugs to rat and human hepatic stellate cells, Hepatology 295 (1999) 1486-1493.
[2] L. Beljaars, P. Olinga, G. Molema, P. De Bleser, A. Geerts, G.M.M. Groothuis, D.K.F. Meijer, and K. Poelstra, Characteristics of the hepatic stellate cell-selective carrier mannose 6-phosphate modified albumin (M6P28-HSA), Liver 215 (2001) 320-328.
[3] R.J. Kok, A.J. Schraa, E.J. Bos, H. E. Moorlag, S.A. Asgeirsdottir, M. Everts, D.K.F. Meijer, and G. Molema, Preparation and functional evaluation of RGD-modified proteins as alpha(v)beta(3) integrin directed therapeutics, Bioconjugate Chemistry 13 (2002) 128-135.
[4] J. Prakash, A. van Loenen-Weemaes, M. Haas, J.H. Proost, D.K.F. Meijer, F. Moolenaar, K. Poelstra, and R.J. Kok, Renal-selective delivery and ACE inhibition by subcutaneously administered captopril-lysozyme. Drug Metabolism and Disposition 33 (2005) 683-688.
[5] A. Geerts, T. Niki, K. Hellemans, D. De Craemer, K. van den Berg, J.-M. Lazou, G. Stange, M. van de Winkel, and P.J. De Bleser, Purification of rat hepatic stellate cells by side scatter-activated cell sorting, Hepatology 27 (1998) 590-598.

## Claims

1. A cell-targeting complex comprising a targeting moiety and a deliverable compound, wherein said targeting moiety and said deliverable compound are joined by means of a (transition) metal ion complex having at least a first reactive moiety for forming a coordination bond with a reactive site of said targeting moiety and having at least a second reactive moiety for forming a coordination bond with a reactive site of said deliverable compound, and wherein said deliverable compound is a therapeutic compound.

2. Cell-targeting complex according to claim 1, wherein said metal ion complex is a platinum complex.

3. Cell-targeting complex according to claim 2, wherein said platinum complex is a *trans*-platinum complex.

4. Cell-targeting complex according to claim 2, wherein said platinum complex is a cis-platinum complex comprising an inert bidentate moiety as a stabilising bridge.

5. Cell-targeting complex according to any one of claims 2-4, wherein said targeting moiety and said deliverable compound comprise one or more sulphur-containing reactive sites and/or one or more nitrogen-containing reactive sites.

6. Cell-targeting complex according to any one of claims 1-5, wherein said targeting moiety is a member of a binding pair.

7. Cell-targeting complex according to any one of claims 1-6, wherein said targeting moiety is an antibody or a receptor ligand.

8. Cell-targeting complex according to according to any one of claims 1 to 6, wherein said targeting moiety is a charge-modified or terminal sugar moiety-modified macromolecular carrier.

9. Cell-targeting complex according to according to claim 8, wherein said terminal sugar moiety-modified macromolecular carrier is a mannose-6-phosphate modified human serum albumin.

10. Cell-targeting complex according to claim 8 or 9, wherein said carrier is modified with peptide moieties that facilitate receptor recognition.

11. Cell-targeting complex according to any one of claims 1-6, wherein said targeting moiety is a low molecular weight protein, preferably selected from the group consisting of lysozyme, alkaline phosphatase, superoxide dismutase, immunoglobulins or parts thereof (e.g. single chain, Fab-fragments or whole IgG), lactoferrin, xanthine oxidase, and TNF α.

12. Cell-targeting complex according to any one of claims 1-11, wherein said deliverable compound is a protein.

13. Cell-targeting complex according to claim 12, wherein said protein is a recombinant protein.

14. Cell-targeting complex according to claim 12 or 13, wherein said protein comprises at least one histidine residue.

15. Cell-targeting complex according to any one of claims 1-14, wherein said deliverable compound is an anti-inflammatory cytokine.

16. Cell-targeting complex according to claim 15, wherein said cytokine is IL-10.

17. Cell-targeting complex according to any one of claims 1-14, wherein said deliverable compound is pentoxifylline (PTX).

18. A pharmaceutical composition comprising a cell-targeting complex according to any one of claims 1-17, and a pharmaceutically acceptable carrier.

19. A cell-targeting complex comprising a targeting moiety and a deliverable compound, wherein said targeting moiety and said deliverable compound are joined by means of a (transition) metal ion complex having at least a first reactive moiety for forming a coordination bond with a reactive site of said targeting moiety and having at least a second reactive moiety for forming a coordination bond with a reactive site of said deliverable compound, for use as a medicament.

20. Use of a (transition) metal ion complex capable of forming coordination bonds for linking a targeting moiety to a deliverable compound.

21. Use according to claim 20, wherein said metal ion complex is a platinum complex.

22. Use according to claim 21, wherein said platinum complex is a *trans-*platinum complex.

23. Use according to claim 21, wherein said platinum complex is a cis-platinum complex comprising an inert bidentate moiety as a stabilising bridge.

24. Use according to any one of claims 20-23, wherein said targeting moiety and said deliverable compound comprise one or more sulphur-containing reactive sites and/or one or more nitrogen containing reactive-sites.

25. Use according to any one of claims 20-24, wherein said deliverable compound is a therapeutic compound.

26. Use according to any one of claims 25, wherein said therapeutic compound is pentoxifylline (PTX).

27. Use according to any one of claims 20-26, wherein said targeting moiety is mannose-6-phosphate modified human serum albumin.

28. Method of coupling a targeting moiety to a therapeutic compound comprising providing a (transition) metal ion complex having at least a first reactive moiety for forming a coordination bond with a reactive site of said targeting moiety and having at least a second reactive moiety for forming a coordination bond with a reactive site of said therapeutic compound and allowing said (transition) metal ion complex to form coordination bonds with said targeting moiety and said therapeutic compound.

29. Method according to claim 28, wherein said metal ion complex is a platinum complex.

30. Method according to claim 29, wherein said platinum complex is a trans-platinum complex.

31. Method according to claim 29, wherein said platinum complex is a cis-platinum complex comprising an inert bidentate moiety as a stabilising bridge.

32. Method according to any one of claims 28-31, wherein said targeting moiety and said deliverable compound comprise one or more sulphur-containing reactive sites and/or one or more nitrogen-containing reactive sites.

33. Method according to any one of claims 28-32, wherein said targeting moiety is a member of a binding pair.

34. Method according to any one of claims 28-33, wherein said targeting moiety is an antibody, a hapten or a receptor ligand.

35. Method according to any one of claims 28-34, wherein said targeting moiety is mannose-6-phosphate modified human serum albumin.

36. Method according to any one of claims 28-35, wherein said therapeutic compound is pentoxifylline (PTX).

37. A method of targeting therapeutic compounds to selected cell populations comprising administering to a subject in need thereof a therapeutically effective amount of a pharmaceutical composition according to claim 18.
